(19) Europäisches Patentamt
European Patent Office
Office européen des brevets

(11) **EP 0 866 853 B1**

(12) **EUROPEAN PATENT SPECIFICATION**

(45) Date of publication and mention
of the grant of the patent:
**03.03.2004 Bulletin 2004/10**

(51) Int Cl.[7]: **C12Q 1/68**

(21) Application number: **96942920.8**

(86) International application number:
**PCT/US1996/019457**

(22) Date of filing: **06.12.1996**

(87) International publication number:
**WO 1997/020918 (12.06.1997 Gazette 1997/25)**

(54) **METHOD OF SCREENING FOR ENZYME ACTIVITY**

VERFAHREN ZUM SCREENING ENZYMATISCHER AKTIVITÄT

PROCEDE DE DETECTION D'UNE ACTIVITE ENZYMATIQUE

(84) Designated Contracting States:
**AT BE CH DE DK ES FI FR GB GR IE IT LI LU MC NL PT SE**

(30) Priority: **07.12.1995 US 8316 P**
**07.12.1995 US 8317 P**
**07.12.1995 US 8311 P**
**22.05.1996 US 651568**
**02.08.1996 US 692002**

(43) Date of publication of application:
**30.09.1998 Bulletin 1998/40**

(60) Divisional application:
**01102857.8 / 1 130 090**

(73) Proprietor: **DIVERSA CORPORATION**
**San Diego, California 92121 (US)**

(72) Inventor: **SHORT, Jay, M.**
**Encinitas, CA 92024 (US)**

(74) Representative: **VOSSIUS & PARTNER**
**Postfach 86 07 67**
**81634 München (DE)**

(56) References cited:
**WO-A-87/03907          WO-A-97/04077**
**US-A- 5 352 778**

• JACOBSEN, C.S.: "Microscale detection of specific bacterial DNA in soil with a magnetic capture-hybridization and PCR amplification assay" APPLIED AND ENVIRONMENTAL MICROBIOLOGY, vol. 61, no. 9, September 1995 (1995-09), pages 3347-3352, XP002110099
• DELONG, E.F.: "Archaea in coastal marine environments" PROCEEDINGS OF THE NATIONAL ACADEMY OF SCIENCES, vol. 89, 1992, pages 5685-5689, XP002110100
• STEELE, D.B.: "Techniques for selection of industrialy improtant microorganisms" ANNUAL REVIEW OF MICROBIOLOGY, vol. 45, - 1991 pages 89-106, XP002110101
• GENE, 1991, Vol. 106, OSUNA et al., "Combinatorial Mutagenesis of Three Major Groove-contacting Residues of EcoRI: Single and Double Amino Acid Replacements Retaining Methyltransferase-sensitive Activity", pages 7-12.
• BIOCHEMISTRY, 1991, Vol. 30, DUBE et al., "Artificial Mutants Generated by the Insertion of Random Oligonucleotides Into the Putative Nucleoside Binding Site of the HSV-1 Thymidine Kinase Gene", pages 11760-11767.
• MICROBIOLOGICA, April 1995, Vol. 18, BURIONI et al., "Engineering Human Monoclonal Antibody Fragments: A Recombinant Enzyme-linked Fab", pages 127-133.
• NUCLEIC ACID RESEARCH, 1995, Vol. 23, No. 10, BORREGO et al., "Combinatorial Libraries by Cassette Mutagenesis", pages 1834-1835.

**Description**

This application is a continuation-in-part of U.S. application serial no. 08/692,002 filed on August 2, 1996 (copending) which is a continuation-in-part of U.S. provisional application no. 60/008,317 which was filed on December 7, 1995 (copending); a continuation-in-part of U.S. application serial no. 08/651,568 filed on May 22, 1996 (copending) which is a continuation-in-part of U.S. provisional application no. 60/008,316 which was filed on December 7, 1995 (copending); and a continuation-in-part of U.S. provisional application no. 60/008,311 which was filed on December 7, 1995 (copending).

The present invention relates to the production and screening of expression libraries for enzyme activity and, more particularly, to obtaining selected DNA from DNA of a microorganism and to screening of an expression library for enzyme activity which is produced from selected DNA, to the directed mutagenesis of DNA and screening of clones containing the mutagenized DNA for resultant specified protein, particularly enzyme, activity(ies) of interest, and to thermostable enzymes, more particularly, the present invention relates to thermostable enzymes which are stable at high temperature and which have improved activity at lower temperatures.

In US 5,352,778 a method for the isolation of recombinant DNA polymerase from archaebacteria has been described. Said method comprises the step of constructing a DNA probe with specificity for one DNA polymerase from one archaebacterium and the use of said probe for the identification and isolation of DNA coding for DNA polymerase from another archaebacterium. Isolated DNA is used according to US 5,352,778 to construct expression vectors in order to produce commercial quantities of the target DNA polymerase.

The method according to the present invention comprises the step of providing a first library with DNA obtained form at least one microorganism prior to the step of creating a DNA subset by using a nucleic acid probe specific for a common enzyme characteristic.

**SUMMARY OF THE INVENTION**

Industry has recognized the need for new enzymes for a wide variety of industrial applications. As a result, a variety of microorganisms have been screened to ascertain whether such microorganisms have a desired enzyme activity. If such microorganisms do have the desired enzyme activity, the enzyme is then recovered from them. The present invention provides a novel approach for obtaining enzymes for further use, for example, for a wide variety of industrial applications, for medical applications, for packaging into kits for use as research reagents, etc.

Thus, in accordance with the present invention, recombinant enzymes are generated from microorganisms and are classified by various enzyme characteristics.

The present invention provides a process for identifying clones having a specified enzyme activity and is described in claim 1.

In a preferred embodiment of this aspect, DNA obtained from at least one microorganism is selected by recovering from the DNA, DNA which specifically binds, such as by hybridization, to a probe DNA sequence. The DNA obtained from the microorganism or microorganisms can be genomic DNA or genomic gene library DNA. One could even use DNA prepared for vector ligation, for instance. The probe may be directly or indirectly bound to a solid phase by which it is separated from the DNA which is not hybridized or otherwise specifically bound to the probe. The process can also include releasing DNA from said probe after recovering said hybridized or otherwise bound DNA and amplifying the DNA so released.

An aspect of the invention provides a process for obtaining an enzyme having a specified enzyme activity derived from a heterogeneous DNA population, which process comprises screening, for the specified enzyme activity, a library of clones containing DNA from the heterogeneous DNA population which have been exposed to directed mutagenesis towards production of the specified enzyme activity.

Another aspect of the invention provides a process for obtaining an enzyme having a specified enzyme activity, which process comprises: screening, for the specified enzyme activity, a library of clones containing DNA from a pool of DNA populations which have been exposed to directed mutagenesis in an attempt to produce in the library of clones DNA encoding an enzyme having one or more desired characteristics, which can be the same or different from the specified enzyme activity. In a preferred embodiment, the DNA pool which is subjected to directed mutagenesis is a pool of DNA which has been selected to encode enzymes having at least one enzyme characteristic, in particular at least one common enzyme activity.

The process of any of these aspects can further comprise, prior to said directed mutagenesis, selectively recovering from the heterogeneous DNA population DNA which comprises DNA sequences coding for enzymes having at least one common characteristic, which can be the same or different from the specified enzyme activity. Preferably, recovering the DNA preparation comprises contacting the DNA population with a specific binding partner, such as a solid phase bound hybridization probe, for at least a portion of the coding sequences. The common characteristic can be, for example, a class of enzyme activity, such as hydrolase activity. DNA is recovered from clones containing DNA from the heterogeneous DNA population which exhibit the class of enzyme activity. Preferably, the directed mutagenesis is site-specific directed mutagenesis. The process of this aspect can further include a step of prescreening the library of clones for an activity, which can be the same or different from the specified enzyme activity, prior to exposing them to directed mutagenesis. This activity can result, for example, from the expression of a protein of interest.

The heterogeneous DNA population from which the DNA library is derived is a complex mixture of DNA, such as is obtained, for example, from an environmental sample. Such samples can contain unculturable, uncultured or cultured multiple or single organisms. These environmental samples can be obtained from, for example, Arctic and Antarctic ice, water or permafrost sources, materials of volcanic origin, materials from soil or plant sources in tropical areas, etc. A variety of known techniques can be applied to enrich the environmental sample for organisms of interest, including differential culturing, sedimentation gradient, affinity matrices, capillary electrophoresis, optical tweezers and fluorescence activated cell sorting. The samples can also be cultures of a single organism.

Thermostable enzymes are enzymes that function at greater than 60°C. Thermostable enzymes are utilized in both industry and biomedical research in assays where certain steps of the assay are performed at significantly increased temperatures. Thermostable enzymes may be obtained from thermophilic organisms found in hot springs, volcanic origin, tropical areas etc. Examples of such organisms, for instance, include prokaryotic microorganisms, such as eubacteria and archaebacteria (Bronneomerier, K. and Staudenbauer, W. L., D.R. Woods (ed), the Clostridia and Biotechnology, Butterworth Publishers, Stoneham, M.A. (1993), among other organisms.

Thermostable enzymes exhibit greater storage life capacity and organic solvent resistance, as compared to their mesophilic counterparts.

There are applications in industry and in research for thermostable enzymes which exhibit enzyme activity at a desired minimum temperature. An example of this occurs in molecular diagnostics wherein reporter molecules must survive long term storage at room temperature or higher or they need to function in unusual environments, and the assays which employ them are performed at room temperature where the activity of thermostable enzymes is generally very low.

Thus, another embodiment of the invention provides a process for providing a thermostable enzyme having improved enzyme activities at lower temperatures, said enzyme being a member selected from the group consisting of an enzyme or a polynucleotide encoding said enzyme comprising:

(a) subjecting to mutagenesis at least one enzyme which is stable at a temperature of at least 60°C; and

(b) screening mutants produced in (a) for a mutated enzyme or polynucleotide encoding a mutated enzyme, which mutated enzyme is stable at a temperature of at least 60°C and which has an enzyme activity at a temperature of less than 50°C and which has activity greater than the enzyme of step (a).

These and other aspects of the present invention will be apparent to those skilled in the art from the teachings herein.

## BRIEF DESCRIPTION OF THE DRAWINGS

Figures 1A and 1B. Figure 1A is a photograph of an agarose gel containing standards and samples a-f described in Example 2. Samples c-f represent DNA recovered from a genomic DNA library using two specific DNA probes and amplified using gene specific primers, as described in Example 2. Figure 1B is also a photograph of an agarose gel containing standards and samples a-f described in Example 2. Samples c-f represent DNA recovered from a genomic DNA library using two specific DNA probes and amplified using vector specific primers, as described in Example 2.

Figure 2 is a photograph of four colony hybridization plates. Plates A and B showed positive clones i.e., colonies which contained DNA prepared in accordance with the present invention, also contained probe sequence. Plates C and D were controls and showed no positive clones.

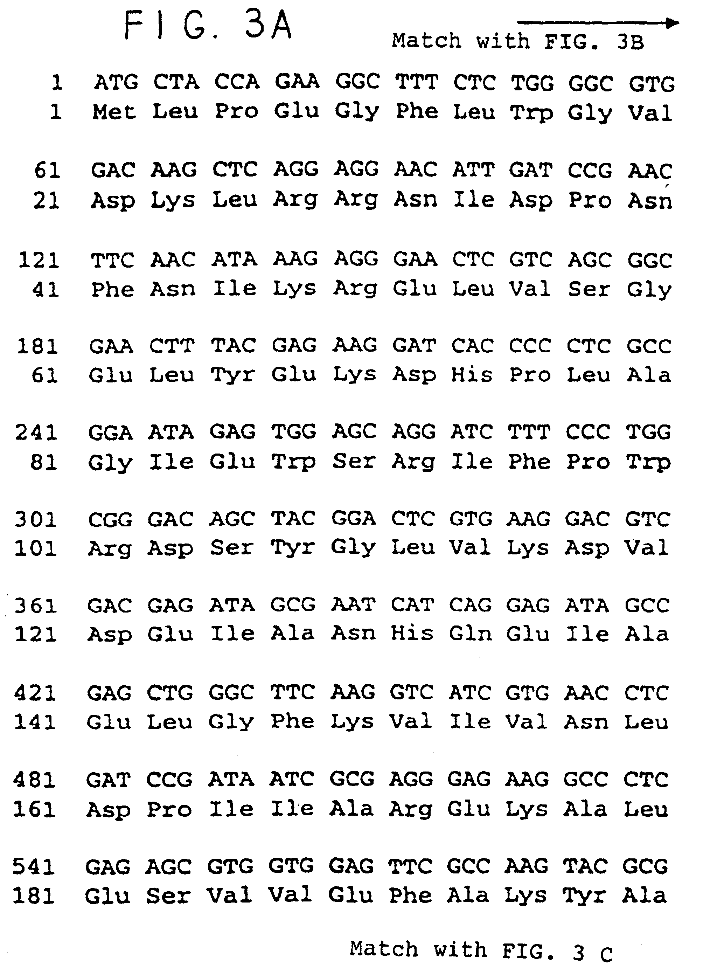

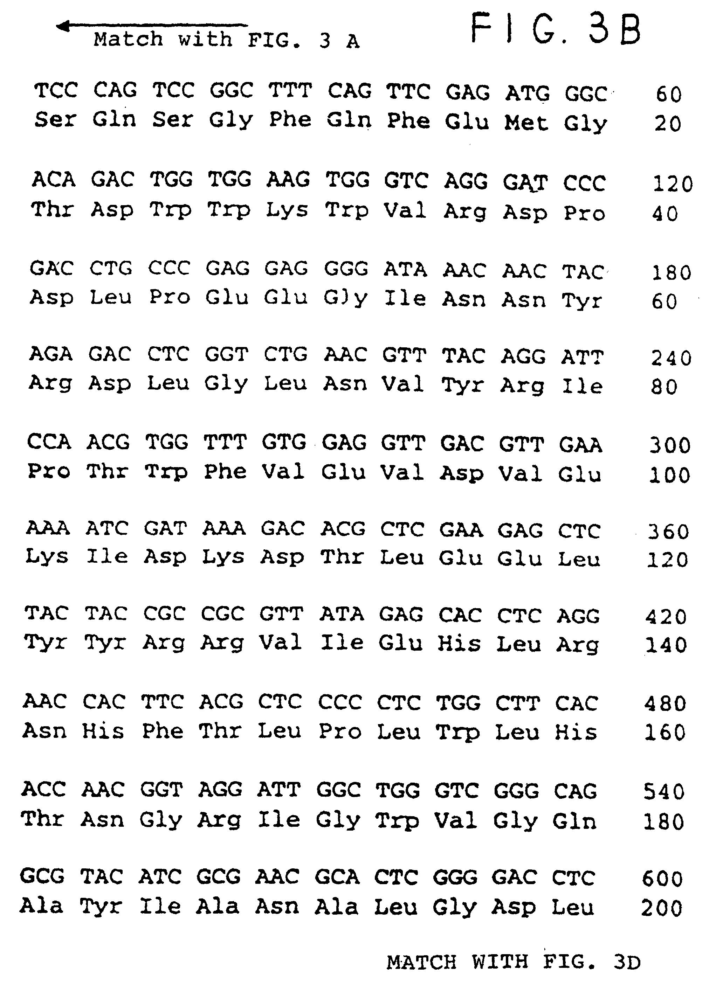

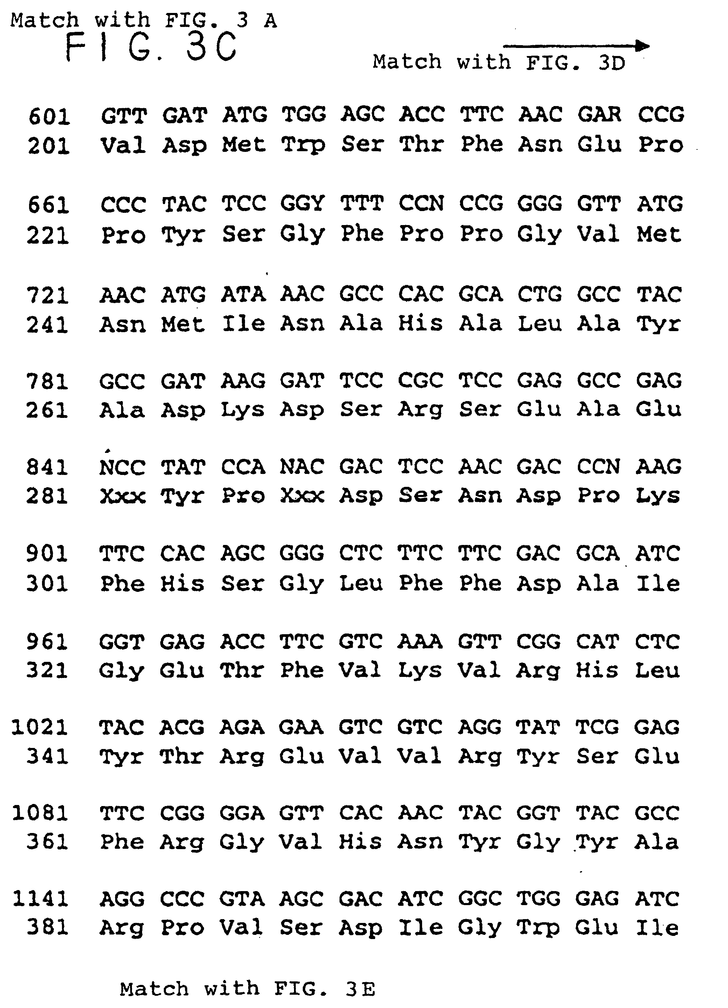

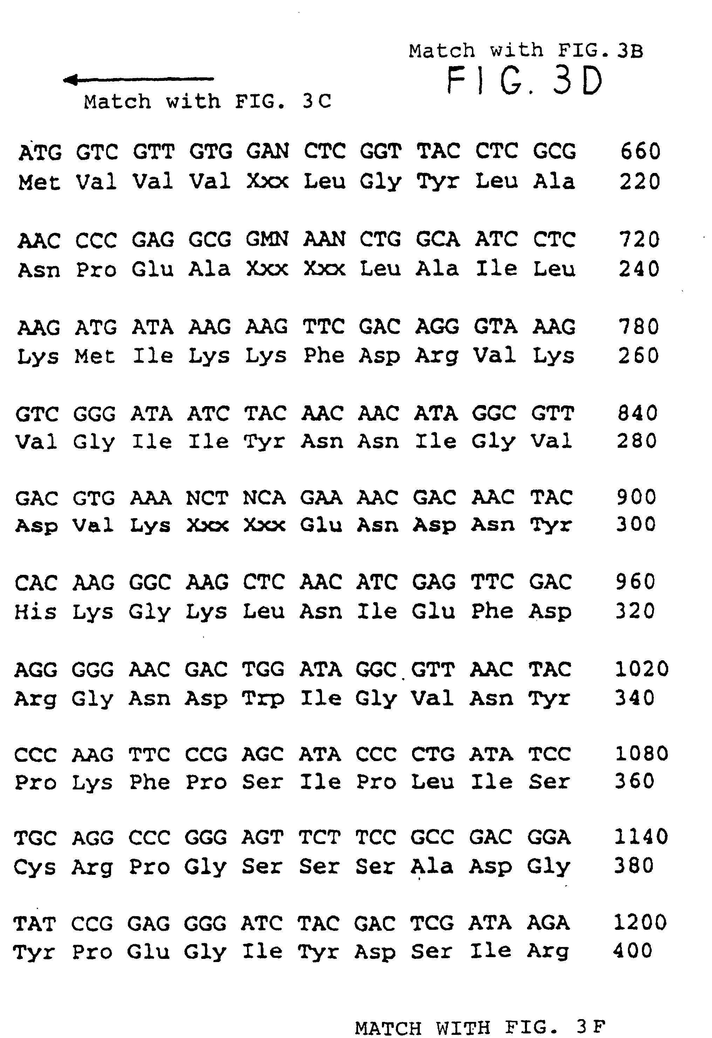

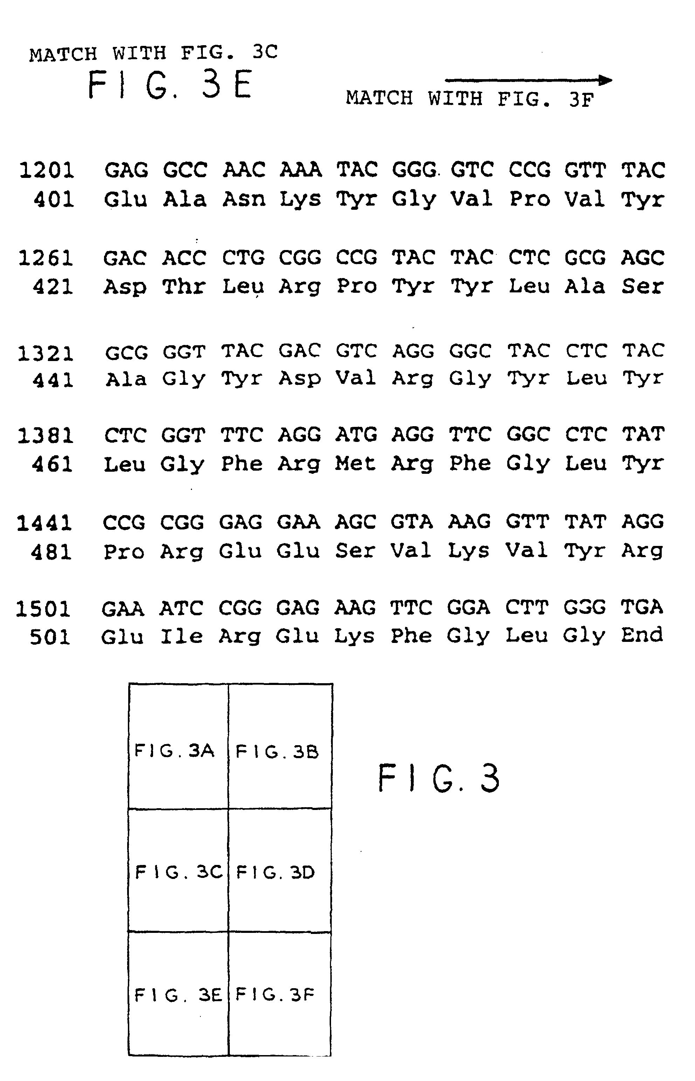

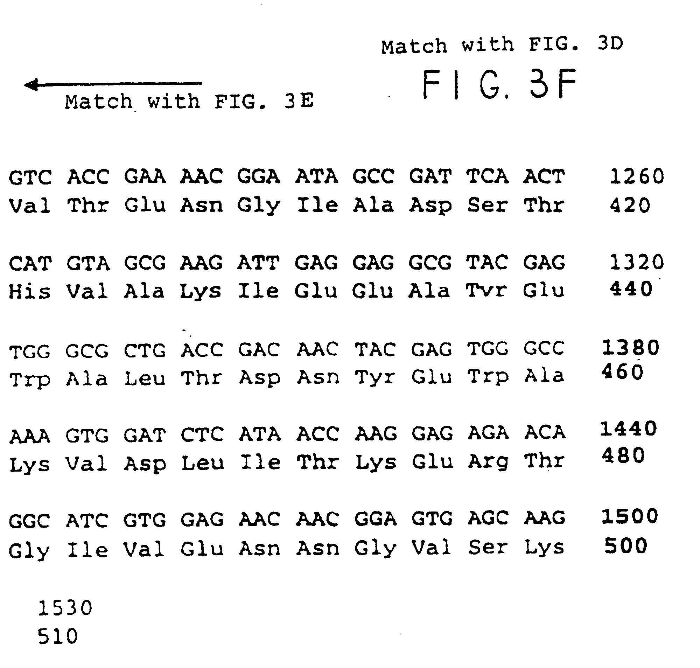

Figure 3 illustrates the full length DNA sequence and corresponding deduced amino acid sequence of Thermococcus 9N2 Beta-glycosidase.

## DETAILED DESCRIPTION OF PREFERRED EMBODIMENTS

The terms "derived" or "isolated" means that material is removed from its original environment (e.g., the natural environment if it is naturally occurring). For example, a naturally-occurring polynucleotide or polypeptide present in a living animal is not isolated, but the same polynucleotide or polypeptide separated from some or all of the coexisting materials in the natural system, is isolated.

The term "error-prone PCR" refers to a process for performing PCR under conditions where the copying fidelity of the DNA polymerase is low, such that a high rate of point mutations is obtained along the entire length of the PCR product. Leung, D.W., *et al.*, Technique, 1:11-15 (1989) and Caldwell, R.C. & Joyce G.F., PCR Methods Applic., 2:28-33 (1992).

The term "oligonucleotide directed mutagenesis" refers to a process which allows for the generation of site-specific mutations in any cloned DNA segment of interest. Reidhaar-Olson, J.F. & Sauer, R.T., *et al.*, Science, 241: 53-57 (1988).

The term "assembly PCR" refers to a process which involves the assembly of a PCR product from a mixture of small DNA fragments. A large number of different PCR reactions occur in parallel in the same vial, with the products of one reaction priming the products of another reaction.

The term "sexual PCR mutagenesis" refers to forced homologous recombination between DNA molecules of different but highly related DNA sequence *in vitro,* caused by random fragmentation of the DNA molecule based on sequence homology, followed by fixation of the crossover by primer extension in a PCR reaction. Stemmer, W.P., PNAS, USA, 91:10747-10751 (1994).

The term "*in vivo* mutagenesis" refers to a process of generating random mutations in any cloned DNA of interest which involves the propogation of the DNA in a strain of *E. coli* that carries mutations in one or more of the DNA repair pathways. These "mutator" strains have a higher random mutation rate than that of a wild-type parent. Propogating the DNA in one of these strains will eventually generate random mutations within the DNA.

The term "cassette mutagenesis" refers to any process for replacing a small region of a double stranded DNA molecule with a synthetic oligonucleotide "cassette" that differs from the native sequence. The oligonucleotide often contains completely and/or partially randomized native sequence.

The term "recursive ensemble mutagenesis" refers to an algorithm for protein engineering (protein mutagenesis) developed to produce diverse populations of phenotypically related mutants whose members differ in amino acid sequence. This method uses a feedback mechanism to control successive rounds of combinatorial cassette mutagenesis. Arkin, A.P. and Youvan, D.C., PNAS, USA, 89:7811-7815 (1992).

The term "exponential ensemble mutagenesis" refers to a process for generating combinatorial libraries with a high percentage of unique and functional mutants, wherein small groups of residues are randomized in parallel to identify, at each altered position, amino acids which lead to functional proteins, Delegrave, S. and Youvan, D.C., Biotechnology Research, 11:1548-1552 (1993); and random and site-directed mutagenesis, Arnold, F.H., Current Opinion in Biotechnology, 4:450-455 (1993). All of the references mentioned above are hereby incorporated by reference in their entirety.

In another aspect to one of the above aspects, the invention provides a process for enzyme activity screening of clones containing selected DNA derived from a microorganism which process comprises:

screening a library for specified enzyme activity, said library including a plurality of clones, said clones having been prepared by recovering from DNA of a microorganism selected DNA, which DNA is selected by hybridization to at least one DNA sequence which is all or a portion of a DNA sequence encoding an enzyme having the specified activity; and
transforming a host with the selected DNA to produce clones which are screened for the specified enzyme activity.

In one embodiment, a DNA library derived from a microorganism is subjected to a selection procedure to select therefrom DNA which hybridizes to one or more probe DNA sequences which is all or a portion of a DNA sequence encoding an enzyme having the specified enzyme activity by:

(a) rendering the double-stranded DNA population into a single-stranded DNA population;
(b) contacting the single-stranded DNA population of (a) with the DNA probe bound to a ligand under conditions permissive of hybridization so as to produce a double-stranded complex of probe and members of the DNA population which hybridize thereto;
(c) contacting the double-stranded complex of (b) with a solid phase specific binding partner for said ligand so as to produce a solid phase complex;
(d) separating the solid phase complex from the single-stranded DNA population of (b);
(e) releasing from the probe the members of the population which had bound to the solid phase bound probe;
(f) forming double-stranded DNA from the members of the population of (e);
(g) introducing the double-stranded DNA of (f) into a suitable host to form a library containing a plurality of clones containing the selected DNA; and
(h) screening the library for the specified enzyme activity.

In another embodiment, a DNA library derived from a microorganism is subjected to a selection procedure to select therefrom double-stranded DNA which hybridizes to one or more probe DNA sequences which is all or a portion of a DNA sequence encoding an enzyme having the specified enzyme activity by:

(a) contacting the double-stranded DNA population with the DNA probe bound to a ligand under conditions permissive of hybridization so as to produce a complex of probe and members of the DNA population which hybridize thereto;

(b) contacting the complex of (a) with a solid phase specific binding partner for said ligand so as to produce a solid phase complex;

(c) separating the solid phase complex from the unbound DNA population of (b);

(d) releasing from the probe the members of the population which had bound to the solid phase bound probe;

(e) introducing the double-stranded DNA of (d) into a suitable host to form a library containing a plurality of clones containing the selected DNA; and

(f) screening the library for the specified enzyme activity.

In another aspect, the process includes a preselection to recover DNA including signal or secretion sequences. In this manner it is possible to select from the DNA population by hybridization as hereinabove described only DNA which includes a signal or secretion sequence. The following paragraphs describe the protocol for this embodiment of the invention, the nature and function of secretion signal sequences in general and a specific exemplary application of such sequences to an assay or selection process.

Another particularly preferred embodiment of this aspect further comprises, after (a) but before (a) above, the steps of:

(*i*). contacting the double-stranded DNA population of (a) with a ligand-bound oligonucleotide probe that is complementary to a secretion signal sequence unique to a given class of proteins under conditions permissive of hybridization to form a double-stranded complex;

(*ii*). contacting the complex of (a *i*) with a solid phase specific binding partner for said ligand so as to produce a solid phase complex;

(*iii*) separating the solid phase complex from the unbound DNA population;

(*iv*) releasing the members of the population which had bound to said solid phase bound probe; and

(*v*) separating the solid phase bound probe from the members of the population which had bound thereto.

The DNA which has been selected and isolated to include a signal sequence is then subjected to the selection procedure hereinabove described to select and isolate therefrom DNA which binds to one or more probe DNA sequences derived from DNA encoding an enzyme(s) having the specified enzyme activity.

The pathways by which proteins are sorted and transported to their proper cellular location are often referred to as protein targeting pathways. One of the most important elements in all of these targeting systems is a short amino acid sequence at the amino terminus of a newly synthesized polypeptide called the signal sequence. This signal sequence directs a protein to its appropriate location in the cell and is removed during transport or when the protein reaches its final destination. Most lysosomal, membrane, or secreted proteins have an amino-terminal signal sequence that marks them for translocation into the lumen of the endoplasmic reticulum. More than 100 signal sequences for proteins in this group have been determined. The sequences vary in length from 13 to 36 amino acid residues.

A phoA expression vector, termed pMG, which, like TaphoA, is useful in identifying genes encoding membrane-spanning sequences or signal peptides. Giladi et al., J. Bacteriol., 175(13):4129-4136, 1993. This cloning system has been modified to facilitate the distinction of outer membrane and periplasmic alkaline phosphatase (AP) fusion proteins from inner membrane AP fusion proteins by transforming pMG recombinants into *E. coli* KS330, the strain utilized in the "blue halo" assay first described by Strauch and Beckwith, *Proc. Nat. Acad. Sci. USA*, *85*:1576-1580, 1988. The pMG/KS330r cloning and screening approach can identify genes encoding proteins with clevable signal peptides and therefore can serve as a first step in the identification of genes encoding polypeptides of interest.

Another embodiment of the invention provides a process for obtaining an enzyme having a specified enzyme activity, derived from a heterogeneous DNA population by screening, for the specified enzyme activity, a library of clones containing DNA from the heterogeneous DNA population which have been exposed to directed mutagenesis towards production of the specified enzyme activity. The process can further comprise, prior to said directed mutagenesis, selectively recovering from the heterogeneous DNA population DNA which comprises DNA sequences coding for a common characteristic, which can be the same or different from the specified enzyme activity. The common characteristic can be, for example, a class of enzyme activity. This involves recovering DNA from clones containing DNA from the heterogeneous DNA population which exhibit the class of enzyme activity.

In this embodiment, recovering the DNA preparation preferably is done by contacting the DNA population with a specific binding partner, such as a solid phase bound hybridization probe, for at least a portion of the coding sequences.

The process of this embodiment can further comprise prescreening said library of clones for an activity, which can be the same or different from the specified enzyme activity, prior to exposing them to directed mutagenesis. The prescreening of said clones can be, for example, for the expression of a protein of interest.

Another embodiment of the invention provides a process for obtaining an enzyme having a specified enzyme activity, which process comprises screening, for the specified enzyme activity, a library of clones containing DNA from a pool of DNA populations which have been exposed to directed mutagenesis, which can be site-specific, in an attempt to produce in the library of clones DNA encoding an enzyme having one or more desired characteristics which can be the same or different from the specified enzyme activity. The process of this embodiment can further include, prior to said directed mutagenesis, selectively recovering from the heterogeneous DNA population DNA which comprises DNA sequences coding for at least one common enzyme characteristic, which can be the same or different from the specified enzyme activity.

In this embodiment, recovering the DNA preparation can comprise contacting the DNA population with a specific binding partner for at least a portion of the coding sequences. The specific binding partner is a solid phase bound hybridization probe. DNA is recovered from clones containing DNA from the heterogeneous DNA population which exhibit the class of enzyme activity.

Applicant has found that it is possible to provide thermostable enzymes which have improved activity at lower temperatures. More particularly, Applicant has found that the activity of thermophilic enzymes can be improved at lower temperatures while maintaining the temperature stability of such enzymes. Further, it has been found that there can be obtained a thermostable enzyme with improved activity at lower temperature by subjecting to mutagenesis a thermostable enzyme or polynucleotide encoding such thermostable enzyme followed by a screening of the resulting mutants to identify a mutated enzyme or a mutated polynucleotide encoding a mutated enzyme, which mutated enzyme retains thermostability and which has an enzyme activity at lower temperatures which is at least two (2) times greater than a corresponding non-mutated enzyme.

The thermostable enzymes and mutated thermostable enzymes are stable at temperatures up to 60°C and preferably are stable at temperatures of up to 70°C and more preferably at temperatures up to 95°C and higher.

Increased activity of mutated thermostable enzymes at lower temperatures is meant to encompass activities which are at least two-fold, preferably at least fourfold, and more preferably at least ten-fold greater than that of the corresponding wild-type enzyme.

Increased enzyme activity at lower temperatures means that enzyme activity is increased at a temperature below 50°C, preferably below 40°C and more preferably below 30°C. Thus, in comparing enzyme activity at a lower temperature between the mutated and non-mutated enzyme, the enzyme activity of the mutated enzyme at defined lower temperatures is at least 2 times greater than the enzyme activity of the corresponding non-mutated enzyme.

Thus, lower temperatures and lower temperature ranges include temperatures which are at least 5°C less than the temperature at which thermostable enzymes are stable, which includes temperatures below 55°C, 50°C, 45°C, 40°C, 35°C, 30°C, 25°C and 20°C, with below 50°C being preferred, and below 40°C being more preferred, and below 30°C (or approximately room temperature) being most preferred.

In accordance with this aspect of the invention, the lower temperature or lower temperature range at which a greater enzyme activity is desired is determined and a thermostable enzyme(s), or polynucleotide encoding such enzyme(s), are subjected to mutagenesis and the resulting mutants are screened to determine mutated enzymes (or polynucleotide encoding mutated enzymes) which retain thermostability and which have a minimum desired increase in enzyme activity at the desired temperature or temperature range.

Thermostable enzymes are enzymes which have activity, i.e. are not degraded, at temperatures above 60°C. Thermostable enzymes also have increased storage life, and high resistance to organic solvents.

Thermostable enzymes may be isolated from thermophilic organisms such as those which are found in elevated temperatures such as in hot springs, volcanic areas and tropical areas. Examples of thermophilic organisms are prokaryotic organisms for example, thermophilic bacteria such as eubacteria and archaebacteria.

The DNA from these thermostable organisms can then be isolated by available techniques that are described in the literature. The IsoQuick® nucleic acid extraction kit (MicroProbe Corporation) is suitable for this purpose.

Alternatively, enzymes not known to have thermostable properties can be screened for such properties by inserting the DNA encoding the enzyme in an expression vector and transforming a suitable host as hereinafter described, such that the enzyme may be expressed and screened for positive thermostable activity.

The isolated DNA encoding a thermostable enzyme is subjected to mutagenesis techniques, with the preferred type of mutagenesis techniques being set forth herein.

As can be seen from the above-defined mutagenesis techniques, the DNA encoding an enzyme having the desired activity may be subject to mutagenesis alone, i.e. as naked DNA, or the DNA may be subjected to mutagenesis after insertion into an appropriate vector as described herein. These techniques are referred to as *in vitro* mutagenesis.

Alternatively, *in vivo* mutagenesis may be performed wherein the DNA is subjected to mutagenesis while it is within a cell or living organism. A preferred example of this technique utilizes the XL1 Red Strain of *E. coli* (Stratagene, Inc.) which has its DNA repair genes, MutH, MutL and MutS, deleted such that many different mutations occur in a short time. Up to 10,000 mutations may take place within a 30 hour time span such that an entire mutated DNA library may be prepared from mutated DNA by procedures known in the art.

After an appropriate amount of time to allow mutations to take place, the mutated DNA is excised from the host cell in the case of in vivo mutagenesis and inserted in another appropriate vector and used to transform a non-mutator host, for example, XL1 Blue strain of *E. coli* after which a mutated DNA library is prepared. In the case of in vitro mutagenesis, if the mutated DNA has previously been inserted in an appropriate expression vector, said vector is then used directly to transform an appropriate non-mutator host for the preparation of a mutated DNA library, if the mutagenized DNA is not in an appropriate expression vector.

A library is prepared for screening by transforming a suitable organism. Hosts, particularly those specifically identified herein as preferred, are transformed by artificial introduction of the vectors containing the mutated DNA by inoculation under conditions conducive for such transformation.

The resultant libraries of transformed clones are then screened for clones which display activity for the enzyme of interest in a phenotypic assay for enzyme activity.

For example, having prepared a multiplicity of clones from DNA mutagenized by one of the techniques described above, such clones are screened for the specific enzyme activity of interest.

For example, the clones containing the mutated DNA are now subject to screening procedures to determine their activity within both higher temperatures and within the desired lower temperature range to identify mutants which have the desired increase in activity within the lower temperature range when compared to the corresponding wild-type thermostable enzyme which is non-mutated.

Positively identified clones, i.e. those which contain mutated DNA sequences which express thermostable enzymes which are thermostable and yet have an increased activity at least two times greater than the corresponding wild-type enzyme at temperatures within the lower temperature range, are isolated and sequenced to identify the DNA sequence. As an example, phosphatase activity at the desired lower temperature ranges may be identified by exposing the clones, and thus the thermostable enzyme and testing its ability to cleave an appropriate substrate.

In Example 7 phosphatase and β-galactosidase activity are measured by comparing the wild-type enzymes to the enzymes subjected to mutagenesis. As can be seen from the results reported there, mutagenesis of a wild-type phosphatase and β-galactosidase thermophilic enzyme produce mutated enzymes which were 3 and 2.5 times more active, respectively, at lower temperatures than the corresponding wild-type enzymes within the lower temperature range of room temperature.

In the case of protein engineering, after subjecting a thermophilic enzyme to mutagenesis, the mutagenized enzyme is screened for the desired activity namely, increased activity at lower temperatures while maintaining activity at the higher temperatures. Any of the known techniques for protein mutagenesis may be employed, with particularly preferred mutagenesis techniques being those discussed above.

The DNA derived from a microorganism(s) is preferably inserted into an appropriate vector (generally a vector containing suitable regulatory sequences for effecting expression) prior to subjecting such DNA to a selection procedure to select and isolate therefrom DNA which hybridizes to DNA derived from DNA encoding an enzyme(s) having the specified enzyme activity.

The microorganisms from which the libraries may be prepared include prokaryotic microorganisms, such as Eubacteria and Archaebacteria, and lower eukaryotic microorganisms such as fungi, some algae and protozoa. The microorganisms may be cultured microorganisms or uncultured microorganisms obtained from environmental samples and such microorganisms may be extremophiles, such as thermophiles, hyperthermophiles, psychrophiles, psychrotrophs, *etc.*

As indicated above, the library may be produced from environmental samples in which case DNA may be recovered without culturing of an organism or the DNA may be recovered from a cultured organism.

Sources of microorganism DNA as a starting material library from which target DNA is obtained are particularly contemplated to include environmental samples, such as microbial samples obtained from Arctic and Antarctic ice, water or permafrost sources, materials of volcanic origin, materials from soil or plant sources in tropical areas, etc. Thus, for example, DNA may be recovered from either a culturable or non-culturable organism and employed to produce an appropriate recombinant expression library for subsequent determination of enzyme activity.

Bacteria and many eukaryotes have a coordinated mechanism for regulating genes whose products are involved in related processes. The genes are clustered, in structures referred to as "gene clusters," on a single chromosome and are transcribed together under the control of a single regulatory sequence, including a single promoter which initiates transcription of the entire cluster. The gene cluster, the promoter, and additional sequences that function in regulation altogether are referred to as an "operon" and can include up to 20 or more genes, usually from 2 to 6 genes. Thus, a gene cluster is a group of adjacent genes that are either identical or related, usually as to their function.

Some gene families consist of identical members. Clustering is a prerequisite for maintaining identity between genes, although clustered genes are not necessarily identical. Gene clusters range from extremes where a duplication is generated to adjacent related genes to cases where hundreds of identical genes lie in a tandem array. Sometimes no significance is discernable in a repetition of a particular gene. A principal example of this is the expressed duplicate insulin genes in some species, whereas a single insulin gene is adequate in other mammalian species.

It is important to further research gene clusters and the extent to which the full length of the cluster is necessary for the expression of the proteins resulting therefrom. Further, gene clusters undergo continual reorganization and, thus, the ability to create heterogeneous libraries of gene clusters from, for example, bacterial or other prokaryote sources is valuable in determining sources of novel proteins, particularly including enzymes such as, for example, the polyketide synthases that are responsible for the synthesis of polyketides having a vast array of useful activities. Other types of proteins that are the product(s) of gene clusters are also contemplated, including, for example, antibiotics, antivirals, antitumor agents and regulatory proteins, such as insulin.

Polyketides are molecules which are an extremely rich source of bioactivities, including antibiotics (such as tetracyclines and erythromycin), anti-cancer agents (daunomycin), immunosuppressants (FK506 and rapamycin) , and veterinary products (monensin). Many polyketides (produced by polyketide synthases) are valuable as therapeutic agents. Polyketide synthases are multifunctional enzymes that catalyze the biosynthesis of a huge variety of carbon chains differing in length and patterns of functionality and cyclization. Polyketide synthase genes fall into gene clusters and at least one type (designated type I) of polyketide synthases have large size genes and enzymes, complicating genetic manipulation and in vitro studies of these genes/proteins.

The ability to select and combine desired components from a library of polyketides and postpolyketide biosynthesis genes for generation of novel polyketides for study is appealing. The method(s) of the present invention make it possible to and facilitate the cloning of novel polyketide synthases, since one can generate gene banks with clones containing large inserts (especially when using the f-factor based vectors), which facilitates cloning of gene clusters.

Preferably, the gene cluster DNA is ligated into a vector, particularly wherein a vector further comprises expression regulatory sequences which can control and regulate the production of a detectable protein or protein-related array activity from the ligated gene clusters. Use of vectors which have an exceptionally large capacity for exogenous DNA introduction are particularly appropriate for use with such gene clusters and are described by way of example herein to include the f-factor (or fertility factor) of *E. coli*. This f-factor of *E. coli* is a plasmid which affects high-frequency transfer of itself during conjugation and is ideal to achieve and stably propagate large DNA fragments, such as gene clusters from mixed microbial samples.

The DNA can then be isolated by available techniques that are described in the literature. The IsoQuick* nucleic acid extraction kit (MicroProbe Corporation) is suitable for this purpose.

The DNA isolated or derived from these microorganisms can preferably be inserted into a vector or a plasmid prior to probing for selected DNA. Such vectors or plasmids are preferably those containing expression regulatory sequences, including promoters, enhancers and the like. Such polynucleotides can be part of a vector and/or a composition and still be isolated, in that such vector or composition is not part of its natural environment. Particularly preferred phage or plasmid and methods for introduction and packaging into them are described in detail in the protocol set forth herein.

The following outlines a general procedure for producing libraries from both culturable and non-culturable organisms, which libraries can be probed to select therefrom DNA sequences which hybridize to specified probe DNA:

**ENVIRONMENTAL SAMPLE**

Obtain Biomass
DNA Isolation (various methods)
Shear DNA (25 gauge needle)
Blunt DNA (Mung Bean Nuclease)
Methylate DNA (*Eco*R I Methylase)
Ligate to *Eco*R I linkers (GGAATTCC)
Cut back linkers (*Eco*R I Restriction Endonuclease)
Size Fractionate (Sucrose Gradient)
Ligate to lambda vector (lambda Zap II and gt11)
Package (*in vitro* lambda packaging extract)
Plate on *E. coli* host and amplify

Clones having an enzyme activity of interest are identified by screening. This screening can be done either by hybridization, to identify the presence of DNA coding for the enzyme of interest or by detection of the enzymatic activity of interest.

The probe DNA used for selectively isolating the target DNA of interest from the DNA derived from at least one microorganism can be a full-length coding region sequence or a partial coding region sequence of DNA for an enzyme of known activity. The original DNA library can be preferably probed using mixtures of probes comprising at least a portion of DNA sequences encoding enzymes having the specified enzyme activity. These probes or probe libraries are preferably single-stranded and the microbial DNA which is probed has preferably been converted into single-stranded form. The probes that are particularly suitable are those derived from DNA encoding enzymes having an activity similar or identical to the specified enzyme activity which is to be screened.

The probe DNA should be at least about 10 bases and preferably at least 15 bases. In one embodiment, the entire coding region may be employed as a probe. Conditions for the hybridization in which target DNA is selectively isolated by the use of at least one DNA probe will be designed to provide a hybridization stringency of at least about 50% sequence identity, more particularly a stringency providing for a sequence identity of at least about 70%, preferably 75%.

Hybridization techniques for probing a microbial DNA library to isolate target DNA of potential interest are well known in the art and any of those which are described in the literature are suitable for use herein, particularly those which use a solid phase-bound, directly or indirectly bound, to probe DNA for separation from the remainder of the DNA derived from the microorganisms. Solution phase hybridizations followed by binding of the probe to a solid phase is preferable.

Preferably the probe DNA is "labeled" with one partner of a specific binding pair (i.e. a ligand) and the other partner of the pair is bound to a solid matrix to provide ease of separation of target from its source. The ligand and specific binding partner can be selected from, in either orientation, the following: (1) an antigen or hapten and an antibody or specific binding fragment thereof; (2) biotin or iminobiotin and avidin or streptavidin; (3) a sugar and a lectin specific therefor; (4) an enzyme and an inhibitor therefor; (5) an apoenzyme and cofactor; (6) complementary homopolymeric oligonucleotides; and (7) a hormone and a receptor therefor. The solid phase is preferably selected from: (1) a glass or polymeric surface; (2) a packed column of polymeric beads; and (3) magnetic or paramagnetic particles.

The library of clones prepared as described above can be screened directly for enzymatic activity without the need for culture expansion, amplification or other supplementary procedures. However, in one preferred embodiment, it is considered desirable to amplify the DNA recovered from the individual clones such as by PCR.

Further, it is optional but desirable to perform an amplification of the target DNA that has been isolated. In this embodiment the target DNA is separated from the probe DNA after isolation. It is then amplified before being used to transform hosts. The double stranded DNA selected to include as at least a portion thereof a predetermined DNA sequence can be rendered single stranded, subjected to amplification and reannealed to provide amplified numbers of selected double stranded DNA. Numerous amplification methodologies are now well known in the art.

The selected DNA is then used for preparing a library for screening by transforming a suitable organism. Hosts, particularly those specifically identified herein as preferred, are transformed by artificial introduction of the vectors containing the target DNA by inoculation under conditions conducive for such transformation. One could transform with double stranded circular or linear DNA or there may also be instances where one would transform with single stranded circular or linear DNA.

The resultant libraries of transformed clones are then screened for clones which display activity for the enzyme of interest in a phenotypic assay for enzyme activity.

Having prepared a multiplicity of clones from DNA selectively isolated from an organism, such clones are screened for a specific enzyme activity and to identify the clones having the specified enzyme characteristics.

The screening for enzyme activity may be effected on individual expression clones or may be initially effected on a mixture of expression clones to ascertain whether or not the mixture has one or more specified enzyme activities. If the mixture has a specified enzyme activity, then the individual clones may be rescreened for such enzyme activity or for a more specific activity. Thus, for example, if a clone mixture has hydrolase activity, then the individual clones may be recovered and screened to determine which of such clones has hydrolase activity.

As representative examples of expression vectors which may be used there may be mentioned viral particles, baculovirus, phage, plasmids, phagemids, cosmids, phosmids, bacterial artificial chromosomes, viral DNA (*e.g.* vaccinia, adenovirus, foul pox virus, pseudorabies and derivatives of SV40), P1-based artificial chromosomes, yeast plasmids, yeast artificial chromosomes, and any other vectors specific for specific hosts of interest (such as bacillus, aspergillus, yeast, *etc.*) Thus, for example, the DNA may be included in any one of a variety of expression vectors for expressing a polypeptide. Such vectors include chromosomal, nonchromosomal and synthetic DNA sequences. Large numbers of suitable vectors are known to those of skill in the art, and are commercially available. The following vectors are provided by way of example; Bacterial: pQE70, pQE60, pQE-9 (Qiagen), psiX174, pBluescript SK, pBluescript KS (Stratagene); pTRC99a, pKK223-3, pKK233-3, pDR540, pRIT2T (Pharmacia); Eukaryotic: pWLNEO, pSV2CAT, pOG44, pXT1, pSG (Stratagene), pSVK3, pBPV, pMSG, pSVLSV40 (Pharmacia). However, any other plasmid or vector may be used as long as they are replicable and viable in the host.

A particularly preferred type of vector for use in the present invention contains an f-factor origin replication. The f-factor (or fertility factor) in *E. coli* is a plasmid which effects high frequency transfer of itself during conjugation and less frequent transfer of the bacterial chromosome itself. A particularly preferred embodiment is to use cloning vectors, referred to as "fosmids" or bacterial artificial chromosome (BAC) vectors. These are derived from *E. coli* f-factor which is able to stably integrate large segments of DNA. When integrated with DNA from a mixed uncultured environmental sample, this makes it possible to achieve large genomic fragments in the form of a stable "environmental DNA library."

The DNA derived from a microorganism(s) may be inserted into the vector by a variety of procedures. In general, the DNA sequence is inserted into an appropriate restriction endonuclease site(s) by procedures known in the art. Such procedures and others are deemed to be within the scope of those skilled in the art.

The DNA sequence in the expression vector is operatively linked to an appropriate expression control sequence(s) (promoter) to direct mRNA synthesis. Particular named bacterial promoters include lacI, lacZ, T3, T7, gpt, lambda $P_R$, $P_L$ and trp. Eukaryotic promoters include CMV immediate early, HSV thymidine kinase, early and late SV40, LTRs from retrovirus, and mouse metallothionein-I. Selection of the appropriate vector and promoter is well within the level of ordinary skill in the art. The expression vector also contains a ribosome binding site for translation initiation and a transcription terminator. The vector may also include appropriate sequences for amplifying expression. Promoter regions can be selected from any desired gene using CAT (chloramphenicol transferase) vectors or other vectors with selectable markers.

In addition, the expression vectors preferably contain one or more selectable marker genes to provide a phenotypic trait for selection of transformed host cells such as dihydrofolate reductase or neomycin resistance for eukaryotic cell culture, or such as tetracycline or ampicillin resistance in *E. coli*.

Generally, recombinant expression vectors will include origins of replication and selectable markers permitting transformation of the host cell, *e.g.*, the ampicillin resistance gene of *E. coli* and *S. cerevisiae* TRP1 gene, and a promoter derived from a highly-expressed gene to direct transcription of a downstream structural sequence. Such promoters can be derived from operons encoding glycolytic enzymes such as 3-phosphoglycerate kinase (PGK), α-factor, acid phosphatase, or heat shock proteins, among others. The heterologous structural sequence is assembled in appropriate phase with translation initiation and termination sequences, and preferably, a leader sequence capable of directing secretion of translated protein into the periplasmic space or extracellular medium.

The DNA selected and isolated as hereinabove described is introduced into a suitable host to prepare a library which is screened for the desired enzyme activity. The selected DNA is preferably already in a vector which includes appropriate control sequences whereby selected DNA which encodes for an enzyme may be expressed, for detection of the desired activity. The host cell can be a higher eukaryotic cell, such as a mammalian cell, or a lower eukaryotic cell, such as a yeast cell, or the host cell can be a prokaryotic cell, such as a bacterial cell. Introduction of the construct into the host cell can be effected by calcium phosphate transfection, DRAB-Dextran mediated transfection, or electroporation (Davis, L., Dibner, M., Battey, I., Basic Methods in Molecular Biology, (1986)).

As representative examples of appropriate hosts, there may be mentioned: bacterial cells, such as *E. coli, Streptomyces, Salmonella typhimurium;* fungal cells, such as yeast; insect cells such as *Drosophila S2* and *Spodoptera Sf9;* animal cells such as CHO, COS or Bowes melanoma; adenoviruses; plant cells, *etc*. The selection of an appropriate host is deemed to be within the scope of those skilled in the art from the teachings herein.

With particular references to various mammalian cell culture systems that can be employed to express recombinant protein, examples of mammalian expression systems include the COS-7 lines of monkey kidney fibroblasts, described by Gluzman, Cell, 23:175 (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 splice, and polyadenylation sites may be used to provide the required nontranscribed genetic elements.

Host cells are genetically engineered (transduced or transformed or transfected) with the vectors. The engineered host cells can be cultured in conventional nutrient media modified as appropriate for activating promoters, selecting transformants or amplifying genes. The culture conditions, such as temperature, pH and the like, are those previously used with the host cell selected for expression, and will be apparent to the ordinarily skilled artisan.

The library may be screened for a specified enzyme activity by procedures known in the art. For example, the enzyme activity may be screened for one or more of the six IUB classes; oxidoreductases, transferases, hydrolases, lyases, isomerases and ligases. The recombinant enzymes which are determined to be positive for one or more of the IUB classes may then be rescreened for a more specific enzyme activity.

Alternatively, the library may be screened for a more specialized enzyme activity. For example, instead of generically screening for hydrolase activity, the library may be screened for a more specialized activity, *i.e.* the type of bond on which the hydrolase acts. Thus, for example, the library may be screened to ascertain those hydrolases which act on one or more specified chemical functionalities, such as: (a) amide (peptide bonds), *i.e.* proteases; (b) ester bonds, *i.e.* esterases and lipases; (c) acetals, *i.e.*, glycosidases etc.

The clones which are identified as having the specified enzyme activity may then be sequenced to identify the DNA sequence encoding an enzyme having the specified activity. Thus, in accordance with the present invention it is possible to isolate and identify: (i) DNA encoding an enzyme having a specified enzyme activity, (ii) enzymes having such activity (including the amino acid sequence thereof) and (iii) produce recombinant enzymes having such activity.

Alternatively, clones found to have the enzymatic activity for which the screen was performed are sequenced and then subjected to directed mutagenesis to develop new enzymes with desired activities or to develop modified enzymes with particularly desired properties that are absent or less pronounced in the wild-type enzyme, such as stability to heat or organic solvents. Any of the known techniques for directed mutagenesis are applicable to the invention. For example, particularly preferred mutagenesis techniques for use in accordance with the invention include those discussed below.

The present invention may be employed for example, to identify or produce new enzymes having, for example, the following activities which may be employed for the following uses:

**1 Lipase/Esterase**

a. Enantioselective hydrolysis of esters (lipids)/thioesters

1) Resolution of racemic mixtures
2) Synthesis of optically active acids or alcohols from *meso*-diesters

b. Selective syntheses

1) Regiospecific hydrolysis of carbohydrate esters
2) Selective hydrolysis of cyclic secondary alcohols

c. Synthesis of optically active esters, lactones, acids, alcohols

1) Transesterification of activated/nonactivated esters
2) Interesterification
3) Optically active lactones from hydroxyesters
4) Regio- and enantioselective ring opening of anhydrides

d. Detergents
e. Fat/Oil conversion
f. Cheese ripening

**2 Protease**

a. Ester/amide synthesis
b. Peptide synthesis
c. Resolution of racemic mixtures of amino acid esters
d. Synthesis of non-natural amino acids
e. Detergents/protein hydrolysis

**3 Glycosidase/Glycosyl transferase**

a. Sugar/polymer synthesis
b. Cleavage of glycosidic linkages to form mono, di-and oligosaccharides
c. Synthesis of complex oligosaccharides
d. Glycoside synthesis using UDP-galactosyl transferase
e. Transglycosylation of disaccharides, glycosyl fluorides, aryl galactosides
f. Glycosyl transfer in oligosaccharide synthesis

g. Diastereoselective cleavage of β-glucosylsulfoxides
h. Asymmetric glycosylations
i. Food processing
j. Paper processing

**4      Phosphatase/Kinase**

a. Synthesis/hydrolysis of phosphate esters

1) Regio-, enantioselective phosphorylation
2) Introduction of phosphate esters
3) Synthesize phospholipid precursors
4) Controlled polynucleotide synthesis

b. Activate biological molecule
c. Selective phosphate bond formation without protecting groups

**5      Mono/Dioxygenase**

a. Direct oxyfunctionalization of unactivated organic substrates
b. Hydroxylation of alkane, aromatics, steroids
c. Epoxidation of alkenes
d. Enantioselective sulphoxidation
e. Regio- and stereoselective Bayer-Villiger oxidations

**6      Haloperoxidase**

a. Oxidative addition of halide ion to nucleophilic sites
b. Addition of hypohalous acids to olefinic bonds
c. Ring cleavage of cyclopropanes
d. Activated aromatic substrates converted to *ortho* and *para* derivatives
e. 1.3 diketones converted to 2-halo-derivatives
f. Heteroatom oxidation of sulfur and nitrogen containing substrates
g. Oxidation of enol acetates, alkynes and activated aromatic rings

**7      Lignin peroxidase/Diarylpropane peroxidase**

a. Oxidative cleavage of C-C bonds
b. Oxidation of benzylic alcohols to aldehydes
c. Hydroxylation of benzylic carbons
d. Phenol dimerization
e. Hydroxylation of double bonds to form diols
f. Cleavage of lignin aldehydes

**8      Epoxide hydrolase**

a. Synthesis of enantiomerically pure bioactive compounds
b. Regio- and enantioselective hydrolysis of epoxide

c. Aromatic and olefinic epoxidation by monooxygenases to form epoxides
d. Resolution of racemic epoxides
e. Hydrolysis of steroid epoxides

**9      Nitrile hydratase/nitrilase**

a. Hydrolysis of aliphatic nitriles to carboxamides
b. Hydrolysis of aromatic, heterocyclic, unsaturated aliphatic nitriles to corresponding acids
c. Hydrolysis of acrylonitrile
d. Production of aromatic and carboxamides, carboxylic acids (nicotinamide, picolinamide, isonicotinamide)
e. Regioselective hydrolysis of acrylic dinitrile
f. $\alpha$-amino acids from $\alpha$-hydroxynitriles

**10      Transaminase**

a. Transfer of amino groups into oxo-acids

**11      Amidase/Acylase**

a. Hydrolysis of amides, amidines, and other C-N bonds
b. Non-natural amino acid resolution and synthesis

The following examples illustrate the invention.

**Example 1** (Reference Example)

**Preparation of a Mammalian DNA Library**

The following outlines the procedures used to generate a gene library from a sample of the exterior surface of a whale bone found at 1240 meters depth in the Santa Catalina Basin during a dive expedition.

**Isolate DNA.**

IsoQuick Procedure as per manufacturer's instructions.

**Shear DNA**

1. Vigorously push and pull DNA through a 25G double-hub needle and 1-cc syringes about 500 times.
2. Check a small amount (0.5 $\mu$g) on a 0.8% agarose gel to make sure the majority of the DNA is in the desired size range (about 3-6 kb).

**Blunt DNA**

1. Add:

| H$_2$O | to a final volume of 405 $\mu$l |
|---|---|
| 45 $\mu$l | 10X Mung Bean Buffer |

(continued)

| 2.0 µl | Mung Bean Nuclease (150 u/µl) |

2. Incubate 37°C, 15 minutes.
3. Phenol/chloroform extract once.
4. Chloroform extract once.
5. Add 1 ml ice cold ethanol to precipitate.
6. Place on ice for 10 minutes.
7. Spin in microfuge, high speed, 30 minutes.
8. Wash with 1 ml 70% ethanol.
9. Spin in microfuge, high speed, 10 minutes and dry .

**Methylate DNA**

1. Gently resuspend DNA in 26 µl TE.
2. Add:

| 4.0 µl | 10X *Eco*R I Methylase Buffer |
| 0.5 µl | SAM (32 mM) |
| 5.0 µl | *Eco*R I Methylase (40 u/µl) |

3. Incubate 37°, 1 hour.

**Insure Blunt Ends**

1. Add to the methylation reaction:

| 5.0 µl | 100 mM MgCl$_2$ |
| 8.0 µl | dNTP mix (2.5 mM of each dGTP, dATP, dTTP, dCTP) |
| 4.0 µl | Klenow (5 u/µl) |

2. Incubate 12°C, 30 minutes.
3. Add 450 µl 1X STE.
4. Phenol/chloroform extract once.
5. Chloroform extract once.
6. Add 1 ml ice cold ethanol to precipitate and place on ice for 10 minutes.
7. Spin in microfuge, high speed, 30 minutes.
8. Wash with 1 ml 70% ethanol.
9. Spin in microfuge, high speed, 10 minutes and dry.

**Linker Ligation**

1. Gently resuspend DNA in 7 µl Tris-EDTA (TE).
2. Add:

| 14 µl | Phosphorylated EcoR I linkers (200 ng/µl) |
| 3.0 µl | 10X Ligation Buffer |
| 3.0 µl | 10 mM rATP |
| 3.0 µl | T4 DNA Ligase (4Wu/µl) |

3. Incubate 4°C, overnight.

**EcoR1 Cutback**

1. Heat kill ligation reaction 68°C, 10 minutes.
2. Add:

| | |
|---|---|
| 237.9 µl | $H_2O$ |
| 30 µl | 10X EcoR I Buffer |
| 2.1 µl | EcoR I Restriction Enzyme (100 u/µl) |

3. Incubate 37°C, 1.5 hours.
4. Add 1.5 µl 0.5 M EDTA.
5. Place on ice.

**Sucrose Gradient (2.2 ml) Size Fractionation**

1. Heat sample to 65°C, 10 minutes.
2. Gently load on 2.2 ml sucrose gradient.
3. Spin in mini-ultracentrifuge, 45K, 20°C, 4 hours (no brake).
4. Collect fractions by puncturing the bottom of the gradient tube with a 20G needle and allowing the sucrose to flow through the needle. Collect the first 20 drops in a Falcon 2059 tube then collect 10 1-drop fractions (labelled 1-10). Each drop is about 60 µl in volume.
5. Run 5 µl of each fraction on a 0.8% agarose gel to check the size.
6. Pool fractions 1-4 (about 10-1.5 kb) and, in a separate tube, pool fractions 5-7 (about 5-0.5 kb).
7. Add 1 ml ice cold ethanol to precipitate and place on ice for 10 minutes.
8. Spin in microfuge, high speed, 30 minutes.
9. Wash with 1 ml 70% ethanol.
10. Spin in microfuge, high speed, 10 minutes and dry.
11. Resuspend each in 10 µl TE buffer.

**Test Ligation to Lambda Arms**

1. Plate assay to get an approximate concentration. Spot 0.5 µl of the sample on agarose containing ethidium bromide along with standards (DNA samples of known concentration). View in UV light and estimate concentration compared to the standards. Fraction 1-4 = >1.0 µg/µl. Fraction 5-7 = 500 ng/µl.
2. Prepare the following ligation reactions (5 µl reactions) and incubate 4°C, overnight:

| Sample | $H_2O$ | 10X Ligase Buffer | 10mM rATP | Lambda arms (gt11 and ZAP) | Insert DNA | T4 DNA Ligase (4 Wu/µ) |
|---|---|---|---|---|---|---|
| Fraction 1-4 | 0.5 µl | 0.5 µl | 0.5 µl | 1.0 µl | 2.0 µl | 0.5 µl |
| Fraction 5-7 | 0.5 µl | 0.5 µl | 0.5 µl | 1.0 µl | 2.0 µl | 0.5 µl |

**Test Package and Plate**

1. Package the ligation reactions following manufacturer's protocol. Package 2.5 µl per packaging extract (2 extracts per ligation).

2. Stop packaging reactions with 500 µl SM buffer and pool packaging that came from the same ligation.

3. Titer 1.0 µl of each on appropriate host ($OD_{600}$ = 1.0) [XLI-Blue MRF for ZAP and Y1088 for gt11]

    Add 200 µl host (in mM $MgSO_4$) to Falcon 2059 tubes

    Inoculate with 1 µl packaged phage

    Incubate 37°C, 15 minutes

    Add about 3 ml 48°C top agar

        [50 ml stock containing 150 µl

        IPTG (0.5M) and 300 µl X-GAL

        (350 mg/ml)]

    Plate on 100mm plates and incubate 37°C, overnight.

4. Efficiency results:

| gt11 | $1.7 \times 10^4$ recombinants with 95% background |
|---|---|
| ZAP II | $4.2 \times 10^4$ recombinants with 66% background |

Contaminants in the DNA sample may have inhibited the enzymatic reactions, though the sucrose gradient and organic extractions may have removed them. Since the DNA sample was precious, an effort was made to "fix" the ends for cloning:

**Re-Blunt DNA**

1. Pool all left over DNA that was not ligated to the lambda arms (Fractions 1-7) and add $H_2O$ to a final volume of 12 µl.

Then add:

| 143 µl | $H_2O$ |
|---|---|
| 20 µl | 10X Buffer 2 (from Stratagene's cDNA Synthesis Kit) |
| 23 µl | Blunting dNTP (from Stratagene's cDNA Synthesis Kit) |
| 2.0 µl | Pfu (from Stratagene"s cDNA Synthesis Kit) |

2. Incubate 72°C, 30 minutes.

3. Phenol/chloroform extract once.

4. Chloroform extract once.

5. Add 20 µL 3M NaOAc and 400 µl ice cold ethanol to precipitate.

6. Place at -20°C, overnight.

7. Spin in microfuge, high speed, 30 minutes.

8. Wash with 1 ml 70% ethanol.

9. Spin in microfuge, high speed, 10 minutes and dry.

**(Do NOT Methylate DNA since it was already methylated in the first round of processing)**

**Adaptor Ligation**

1. Gently resuspend DNA in 8 µl EcoR I adaptors (from Stratagene's cDNA Synthesis Kit).

2. Add:

| 1.0 µl | 10X Ligation Buffer |
|---|---|
| 1.0 µl | 10 mM rATP |
| 1.0 µl | T4 DNA Ligase (4Wu/µl) |

3. Incubate 4°C, 2 days.

**(Do NOT cutback since using ADAPTORS this time. Instead, need to phosphorylate)**

**Phosphorylate Adaptors**

    1. Heat kill ligation reaction 70°C, 30 minutes.
        Add:

| | |
|---|---|
| 1.0 µl | 10X Ligation Buffer |
| 2.0 µl | 10mM rATF |
| 6.0 µl | $H_2O$ |
| 1.0 µl | PNK (from Stratagene's cDNA Synthesis Kit). |

    3. Incubate 37°C, 30 minutes.
    4. Add 31 µl $H_2O$ and 5 µl 10X STE.
    5. Size fractionate on a Sephacryl S-500 spin column (pool fractions 1-3).
    6. Phenol/chloroform extract once.
    7. Chloroform extract once.
    8. Add ice cold ethanol to precipitate.
    9. Place on ice, 10 minutes.
    10. Spin in microfuge, high speed, 30 minutes.
    11. Wash with 1 ml 70% ethanol.
    12. Spin in microfuge, high speed, 10 minutes and dry.
    13. Resuspend in 10.5 µl TE buffer.

Do not plate assay. Instead, ligate directly to arms as above except use 2.5 µl of DNA and no water.

**Package and titer as above.**

Efficiency results:

| | |
|---|---|
| *gt*11 | $2.5 \times 10^6$ recombinants with 2.5% background |
| ZAP II | $9.6 \times 10^5$ recombinants with 0% background |

**Amplification of Libraries (5.0 x $10^5$ recombinants from each library)**

    1. Add 3.0 ml host cells ($OD_{600}$=1.0) to two 50 ml conical tube.
    2. Inoculate with $2.5 \times 10^5$ pfu per conical tube.
    3. Incubate 37°C, 20 minutes.
    4. Add top agar to each tube to a final volume of 45 ml.
    5. Plate the tube across five 150 mm plates.
    6. Incubate 37°C, 6-8 hours or until plaques are about pin-head in size.
    7. Overlay with 8-10 ml SM Buffer and place at 4°C overnight (with gentle rocking if possible).

**Harvest Phage**

    1. Recover phage suspension by pouring the SM buffer off each plate into a 50-ml conical tube.
    2. Add 3 ml chloroform, shake vigorously and incubate at room temperature, 15 minutes.
    3. Centrifuge at 2K rpm, 10 minutes to remove cell debris.
    4. Pour supernatant into a sterile flask, add 500 µl chloroform.

5. Store at 4°C.

**Titer Amplified Library**

1. Make serial dilutions:

$10^{-5}$ = 1 μl amplified phage in 1 ml SM Buffer
$10^{-6}$ = 1 μl of the $10^{-3}$ dilution in 1 ml SM Buffer

2. Add 200 μl host (in 10 mM $MgSO_4$) to two tubes.
3. Inoculate one with 10 μl $10^{-6}$ dilution ($10^{-5}$).
4. Inoculate the other with 1 μl $10^{-6}$ dilution ($10^{-6}$).
5. Incubate 37°C, 15 minutes.
6. Add about 3 ml 48°C top agar.
    [50 ml stock containing 150 μl IPTG
    (0.5M) and 375 μl X-GAL (350 mg/ml)]
7. Plate on 100 mm plates and incubate 37°C, overnight.
8. Results:

| | |
|---|---|
| gt11 | 1.7 x $10^{11}$/ml |
| ZAP II | 2.0 x $10^{10}$/ml |

## Example 2

### Construction of a Stable, Large Insert DNA Library of Picoplankton Genomic DNA

**Cell collection and preparation of DNA.** Agarose plugs containing concentrated picoplankton cells were prepared from samples collected on an oceanographic cruise from Newport, Oregon to Honolulu, Hawaii. Seawater (30 liters) was collected in Niskin bottles, screened through 10 μm Nitex, and concentrated by hollow fiber filtration (Amicon DC10) through 30,000 MW cutoff polyfulfone filters. The concentrated bacterioplankton cells were collected on a 0.22 μm, 47 mm Durapore filter, and resuspended in 1 ml of 2X STE buffer (1M NaCl, 0.1M EDTA, 10 mM Tris, pH 8.0) to a final density of approximately 1 x $10^{10}$ cells per ml. The cell suspension was mixed with one volume of 1% molten Seaplaque LMP agarose (FMC) cooled to 40°C, and then immediately drawn into a 1 ml syringe. The syringe was sealed with parafilm and placed on ice for 10 min. The cell-containing agarose plug was extruded into 10 ml of Lysis Buffer (10mM Tris pH 8.0, 50 mM NaCl, 0.1M EDTA, 1% Sarkosyl, 0.2% sodium deoxycholate, 1 mg/ml lysozyme) and incubated at 37°C for one hour. The agarose plug was then transferred to 40 mls of ESP Buffer (1% Sarkosyl, 1 mg/ml proteinase K, in 0.5M EDTA), and incubated at 55°C for 16 hours. The solution was decanted and replaced with fresh ESP Buffer, and incubated at 55°C for an additional hour. The agarose plugs were then placed in 50 mM EDTA and stored at 4°C shipboard for the duration of the oceanographic cruise.

One slice of an agarose plug (72 μl) prepared from a sample collected off the Oregon coast was dialyzed overnight at 4°C against 1 mL of buffer A (100mM NaCl, 10mM Bis Tris Propane-HCl, 100 μg/ml acetylated BSA: pH 7.0 @ 25°C) in a 2 mL microcentrifuge tube. The solution was replaced with 250 μl of fresh buffer A containing 10 mM $MgCl_2$ and 1 mM DTT and incubated on a rocking platform for 1 hr at room temperature. The solution was then changed to 250 μl of the same buffer containing 4U of Sau3A1 (NEB), equilibrated to 37°C in a water bath, and then incubated on a rocking platform in a 37°C incubator for 45 min. The plug was transferred to a 1.5 ml microcentrifuge tube and incubated at 68°C for 30 min to inactivate the enzyme and to melt the agarose. The agarose was digested and the DNA dephosphorylased using Gelase and HK-phosphatase (Epicentre), respectively, according to the manufacturer's recommendations. Protein was removed by gentle phenol/chloroform extraction and the DNA was ethanol precipitated, pelleted, and then washed with 70% ethanol. This partially digested DNA was resuspended in sterile $H_2O$ to a concentration of 2.5 ng/μl for ligation to the pFOS1 vector.

PCR amplification results from several of the agarose plugs (data not shown) indicated the presence of significant amounts of archaeal DNA. Quantitative hybridization experiments using rRNA extracted from one sample, collected at 200 m of depth off the Oregon Coast, indicated that planktonic archaea in (this assemblage comprised approximately 4.7% of the total picoplankton biomass (this sample corresponds to "PACI"-200 m in Table 1 of DeLong et al., high abundance of Archaea in Antarctic marine picoplankton, *Nature*, 371:695-698, 1994). Results from archaeal-biased rDNA PCR amplification performed on agarose plug lysates confirmed the presence of relatively large amounts of archaeal DNA in this sample. Agarose plugs prepared from this picoplankton sample were chosen for subsequent fosmid library preparation. Each 1 ml agarose plug from this site contained approximately $7.5 \times 10^5$ cells, therefore approximately $5.4 \times 10^5$ cells were present in the 72 µl slice used in the preparation of the partially digested DNA.

Vector arms were prepared from pFOS1 as described (Kim *et al*., Stable propagation of casmid sized human DNA inserts in an F factor based vector, *Nucl. Acids Res., 20:*10832-10835, *1992*). Briefly, the plasmid was completely digested with AstII, dephosphorylated with HK phosphatase, and then digested with BamHI to generate two arms, each of which contained a *cos* site in the proper orientation for cloning and packaging ligated DNA between 35-45 kbp. The partially digested picoplankton DNA was ligated overnight to the PFOS1 arms in a 15 µl ligation reaction containing 25 ng each of vector and insert and 1U of T4 DNA ligase (Boehringer-Mannheim). The ligated DNA in four microliters of this reaction was *in vitro* packaged using the Gigapack XL packaging system (Stratagene), the fosmid particles transfected to *E. coli* strain DH10B (BRL), and the cells spread onto $LB_{cm15}$ plates. The resultant fosmid clones were picked into 96-well microliter dishes containing $LB_{cm15}$ supplemented with 7% glycerol. Recombinant fosmids, each containing ca. 40 kb of picoplankton DNA insert, yielded a library of 3.552 fosmid clones, containing approximately $1.4 \times 10^8$ base pairs of cloned DNA. All of the clones examined contained inserts ranging from 38 to 42 kbp. This library was stored frozen at -80°C for later analysis.

**Example 3**

**Hybridization Selection and Production of Expression Library**

Starting with a plasmid library prepared as described in Example 1, hybridization selection and preparation of the expression library were performed according to the protocol described in this example. The library can contain DNA from isolated microorganisms, enriched cultures or environmental samples.

Single-stranded DNA is made in one of two ways: 1) The plasmid library can be grown and the double-stranded plasmid DNA isolated. The double-stranded DNA is made single-stranded using F1 gene II protein and Exonuclease III. The gene II protein nicks the double-stranded plasmids at the F1 origin and the Exo III digests away the nicked strand leaving a single-stranded circle. This method is used by Life Technologies in their GeneTrapper™ kit; 2) the second method involves the use of a helper phage to "rescue" one of the strands of the double-stranded plasmids. The plasmid library is grown in a small overnight culture. A small aliquot of this is mixed with VCS-M13 helper phage and again grown overnight. The next morning the phagemids (virus particles containing single-stranded DNA) are recovered from the media and used in the following protocol.

**PROTOCOL**

1. Six samples of 4 µg of rescued, single-stranded DNA from library #17 were prepared in 3X SSC buffer. Final reaction volumes were 30 µl.

2. To these solutions was added one of the following:

    a) nothing
    b) 100 ng of biotinylated probe from an unrelated sequence
    c,d) 100 ng of biotinylated probe from organism #13 DNA polymerase gene
    e,f) 100 ng of biotinylated probe from organism #17 DNA polymerase gene

Biotinylated probes were prepared by PCR amplification of fragments of -1300 bp in length coding for a portion of the DNA polymerase gene of these organisms. The amplification products were made using biotinylated dUTP in the amplification mix. This modified nucleotide is incorporated throughout the DNA during synthesis. Unincorporated nucleotides were removed using the QIAGEN PCR Cleanup kit.

3. These mixtures were denatured by heating to 95°C for 2 minutes.

4. Hybridization was performed for 90 minutes at 70°C for samples a, b, d and f. Samples c and e were hybridized at 60°C.

5. 50 µl of washed and blocked MPG beads were added and mixed to each sample. These mixtures were agitated every 5 minutes for a total of 30 minutes. MPG beads are sent at 1 mg/ml in buffer containing preservative so 6 sets of 100 μl were washed 2 times in 3X SSC and resuspended in 60 μl of 3X SSC containing 100 μg of sonicated salmon sperm DNA.

6. The DNA/bead mixtures were washed 2 times at room temperature in 0.1X SSC/0.1% SDS, 2 times at 42°C in 0.1X SSC/0.1% SDS for 10 minutes each and 1 additional wash at room temperature with 3X SSC.

7. The bound DNA was eluted by heating the beads to 70°C for 15 minutes in 50 μl TE.

8. Dilutions of the eluted DNAs were made and PCR amplification was performed with either gene specific primers or vectors specific primers. Dilutions of the library DNA were used as standards.

9. The DNA inserts contained within the DNA were amplified by PCR using vector specific primers. These inserts were cloned using the TA Cloning system (Invitrogen).

10. Duplicates of 92 white colonies and 4 blue colonies from samples d and f were grown overnight and colony lifts were prepared for Southern blotting.

11. The digoxigenin system from Boehringer Mannheim was used to probe the colonies using the organism #17 probe.

### RESULTS

### PCR Quantitation

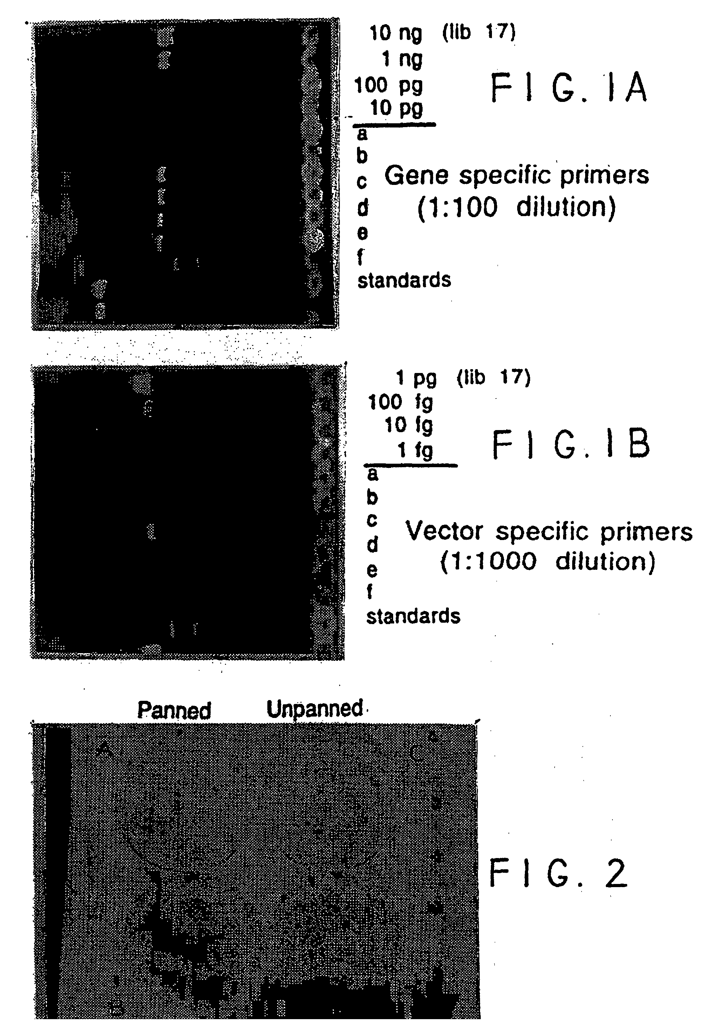

Figures 1A and 1B. Figure 1A is a photograph of the autoradiogram resulting from the Southern hybridization agarose gel electrophoresis columns of DNA from sample solutions a-f in Example 2, when hybridized with gene specific primers. Figure 1B is a photograph of the autoradiogram resulting from the Southern hybridization agarose gel electrophoresis columns of DNA from sample solutions a-f in Example 2, when hybridized with vector specific primers.

The gene specific DNA amplifications of samples a and b demonstrate that non-specific binding to the beads is minimal. The amount of DNA bound under the other conditions results in the following estimates of enrichment.

|  | gene specific equivalent | total | enrichment |
|---|---|---|---|
| c | 50 ng | 100 pg | 500X |
| d | 50 ng | 30 pg | 1667X |
| e | 20 ng | 50 pg | 400X |
| f | 20 ng | 20 pg | 1000X |

### Colony Hybridization

Figure 2 is a photograph of four colony hybridization plates resulting from Plates A and B showing positive clones i.e., colonies containing sequences contained in the probe and which contain DNA from a library prepared in accordance with the invention. Plates C and D were controls and showed no positive clones.

Seven of 92 colonies from the panned sample were positive for sequences contained in the probe. No positive clones were found in the unpanned sample.

### Example 4

### Enzymatic Activity Assay

The following is a representative example of a procedure for screening an expression library, prepared in accordance with Example 1, for hydrolase activity.

Plates of the library prepared as described in Example 1 are used to multiply inoculate a single plate containing 200 μL of LB Amp/Meth, glycerol in each well. This step is performed using the High Density Replicating Tool (HDRT) of the Beckman Biomek with a 1% bleach, water, isopropanol, air-dry sterilization cycle between each inoculation. The single plate is grown for 2h at 37°C and is then used to inoculate two white 96-well Dynatech microtiter daughter plates containing 250 μL of LB Amp/Meth, glycerol in each well. The original single plate is incubated at 37°C for 18h, then stored at -80°C. The two condensed daughter plates are incubated at 37°C also for 18 h. The condensed daughter plates are then heated at 70°C for 45 min. to kill the cells and inactivate the host *E. coli* enzymes. A stock solution of 5mg/mL morphourea phenylalanyl-7-amino-4-trifluoromethyl coumarin (MuPheAFC, the 'substrate') in DMSO is diluted to 600 μM with 50 mM pH 7.5 Hepes buffer containing 0.6 mg/mL of the detergent dodecyl maltoside.

MuPheAFC

Fifty μL of the 600 μM MuPheAFC solution is added to each of the wells of the white condensed plates with one 100 μL mix cycle using the Biomek to yield a final concentration of substrate of ∼ 100 μM. The fluorescence values are recorded (excitation = 400 nm, emission = 505 nm) on a plate reading fluorometer immediately after addition of the substrate (t=0). The plate is incubated at 70°C for 100 min, then allowed to cool to ambient temperature for 15 additional minutes. The fluorescence values are recorded again (t=100). The values at t=0 are subtracted from the values at t=100 to determine if an active clone is present.

The data will indicate whether one of the clones in a particular well is hydrolyzing the substrate. In order to determine the individual clone which carries the activity, the source library plates are thawed and the individual clones are used to singly inoculate a new plate containing LB Amp/Meth, glycerol. As above, the plate is incubated at 37°C to grow the cells, heated at 70°C to inactivate the host enzymes, and 50 μL of 600 μM MuPheAFC is added using the Biomek.

After addition of the substrate the t=0 fluorescence values are recorded, the plate is incubated at 70°C, and the t=100 min. values are recorded as above. These data indicate which plate the active clone is in.

The enantioselectivity value, E, for the substrate is determined according to the equation below:

$$E = \frac{\ln[(1-c(1+ee_p)]}{\ln[(1-c(1-ee_p)]}$$

where $ee_p$ = the enantiomeric excess (ee) of the hydrolyzed product and c = the percent conversion of the reaction. See Wong and Whitesides, Enzymes in Synthetic Organic Chemistry, 1994, Elsevier, Tarrytown, New York, pp. 9-12.

The enantiomeric excess is determined by either chiral high performance liquid chromatography (HPLC) or chiral capillary electrophoresis (CE). Assays are performed as follows: two hundred μL of the appropriate buffer is added to each well of a 96-well white microtiter plate, followed by 50 μL of partially or completely purified enzyme solution; 50 μL of substrate is added and the increase in fluorescence monitored versus time until 50% of the substrate is consumed or the reaction stops, whichever comes first.

## Example 5

### Mutagenesis of positive Enzyme Activity Clones

Mutagenesis was performed on two different enzymes (alkaline phosphatase and β-glycosidase), using the two different strategies described here, to generate new enzymes which exhibit a higher degree of activity than the wild-type enzymes.

### Alkaline Phosphatase

The XL1-Red strain (Stratagene) was transformed with genomic clone 27a3a (in plasmid pBluescript) encoding the alkaline phosphatase gene from the organism OC9a according to the manufacturer's protocol. A 5ml culture of LB + 0.1 mg/ml ampicillin was inoculated with 200μl of the transformation. The culture was allowed to grow at 37°C for 30 hours. A miniprep was then performed on the culture, and screening was performed by transforming 2μl of the resulting DNA into XL-1 Blue cells (Stratagene) according to the manufacturer's protocol and following procedure outlined below (after "Transform XL1 Blue cells). The mutated OC9a phosphatase took 10 minutes to develop color and the wild type enzyme took 30 minutes to develop color in the screening assay.

### Standard Alkaline Phosphatase Screening Assay

Transform XL1 Red strain → Inoculate 5ml LB/amp culture with 200μl transformation and incubate at 37°C for 30 hours → Miniprep DNA → Transform XL1 Blue cells → Plate on LB/amp plates → Lift colonies with Duralon UV (Stratagene) or HATF (Millipore) membranes → Lyse in chloroform vapors for 30 seconds → Heat kill for 30 minutes at 85°C → Develop filter at room temperature in BCIP buffer → Watch as filter develops and identify and pick fastest developing colonies ("positives") → Restreak "positives" onto a BCIP plate

**BCIP Buffer:**

20mm CAPS pH 9.0
1mm $MgCl_2$
0.01 mm $ZnCl_2$
0.1 mg/ml BCIP

**Beta-Glycosidase**

This protocol was used to mutagenize Thermococcus 9N2 Beta-Glycosidase.

**PCR Reaction**

2 microliters dNTP's (10mM Stocks)
10 microliters 10xPCR Buffer
.5 microliters Vector DNA-31G1A-100 nanograms
20 microliters 3' Primer (100 pmol)
20 microliters 5' Primer (100 pmol)
16 microliters MnCl $4H_2O$ (1.25mM Stock)
24.5 microliters $H_2O$
1 microliter *Taq* Polymerase (5.0 Units)
100 microliters total

**Reaction Cycle**

95°C 15 seconds
58°C 30 seconds
72°C 90 seconds
25 cycles (10 minute extension at 72°C-4°C incubation)

Run 5 microliters on a 1% agarose gel to check the reaction.
Purify on a Qiaquick column (Qiagen).
Resuspend in 50 microliters $H_2O$.

**Restriction Digest**

25 microliters purified PCR product
10 microliters NEB Buffer #2
3 microliters Kpn I (1OU/microliter)
3 microliters EcoR1 (20U/microliter)
59 microliters $H_2O$

Cut for 2 hours at 37°C.
Purify on a Qiaquick column (Qiagen).
Elute with 35 microliters $H_2O$.

**Ligation**

10 microliters Digested PCR product
5 microliters Vector (cut with EcoRI/KpnI and
phosphatased with shrimp alkaline phosphatase
4 microliters 5x Ligation Buffer
1 microliter T4 DNA Ligase (BRL)

Ligate overnight.
Transform into M15pREP4 cells using electroporation.
Plate 100 or 200 microliters onto LB amp meth kan plates, grow overnight at 37 degrees celsius.

**Beta-Glycosidase Assay**

Perform glycosidase assay to screen for mutants as follows. The filter assay uses buffer Z (see recipe below) containing 1 mg/ml of the substrate 5-bromo-4-chloro-3-indolyl-β-o-glucopyranoside (XGLU) (Diagnostic Chemicals Limited or Sigma).

<u>Z-Buffer:</u> (referenced in Miller, J.H. (1992) A Short Course in Bacterial Genetics, p. 445.)
per liter:

| | |
|---|---|
| $Na_2HPO_4$-$7H_2O$ | 16.1 g |
| $NaH_2PO_4$-$H_2O$ | 5.5 g |
| KCl | 0.75 g |
| $MgSO_4$-$7H_2O$ | 0.246 g |
| β-mercaptoethanol | 2.7 ml |

Adjust pH to 7.0

(1) Perform colony lifts using Millipore HATF membrane filters.

(2) Lyse colonies with chloroform vapor in 150 mm glass petri dishes.

(3) Transfer filters to 100 mm glass petri dishes containing a piece of Whatman 3MM filter paper saturated with Z buffer containing 1 mg/ml XGLU. After transferring filter bearing lysed colonies to the glass petri dish, maintain dish at room temperature.

(4) "Positives" were observed as blue spots on the filter membranes ("positives" are spots which appear early). Use the following filter rescue technique to retrieve plasmid from lysed positive colony. Use pasteur pipette (or glass capillary tube) to core blue spots on the filter membrane. Place the small filter disk in an Epp tube containing 20 μl water. Incubate the Epp tube at 75°C for 5 minutes followed by vortexing to elute plasmid DNA off filter. Transform this DNA into electrocompetent *E. coli* cells. Repeat filter-lift assay on transformation plates to identify "positives." Return transformation plates to 37°C incubator after filter lift to regenerate colonies. Inoculate 3 ml LBamp liquid with repurified positives and incubate at 37°C overnight. Isolate plasmid DNA from these cultures and sequence plasmid insert.

**Example 7**

**Directed Mutagenesis of Positive Enzyme Activity Clones**

Directed mutagenesis was performed on two different enzymes (alkaline phosphatase and β-glycosidase), using the two different strategies described here, to generate new enzymes which exhibit a higher degree of activity at lower temperatures than the wild-type enzymes.

**Alkaline Phosphatase**

The XL1-Red strain (Stratagene) was transformed with DNA encoding an alkaline phosphatase (in plasmid pBluescript) from the organism OC9a according to the manufacturer's protocol. A 5ml culture of LB + 0.1 mg/ml ampicillin was inoculated with 200µl of the transformation. The culture was allowed to grow at 37°C for 30 hours. A miniprep was then performed on the culture, and screening was performed by transforming 2µl of the resulting DNA into XL-1 Blue cells (Stratagene) according to the manufacturer's protocol.

**Standard Alkaline Phosphatase Screening Assay**

→ Plate on LB/amp plates → Lift colonies with Duralon UV (Stratagene) or HATF (Millipore) membranes → Lyse in chloroform vapors for 30 seconds → Heat kill for 30 minutes at 85°C → Develop filter at room temperature in BCIP buffer → Watch as filter develops and identify and pick fastest developing colonies ("positives") → Restreak "positives" onto a BCIP plate.

**BCIP Buffer:**

20mm CAPS pH 9.0
1mm $MgCl_2$
0.01 mm $ZnCl_2$
0.1 mg/ml BCIP

The mutated OC9a phosphatase took 10 minutes to develop color and the wild type enzyme took 30 minutes to develop color in the screening assay.

**Beta-Glycosidase**

This protocol was used to mutagenize DNA encoding Thermococcus 9N2 Beta-Glycosidase. This DNA sequence is set forth in Figure 1.

**PCR**

2 microliters dNTP's (10mM Stocks)
10 microliters 10xPCR Buffer
.5 microliters pBluescript vector containing Beta-glycosidase DNA (100 nanograms)
20 microliters 3' Primer (100 pmol)
20 microliters 5' Primer (100 pmol)
16 microliters MnCl $4H_2O$ (1.25mM Stock)
24.5 microliters $H_2O$
1 microliter *Taq* Polymerase (5.0 Units)
100 microliters total

**Reaction Cycle**

95°C 15 seconds
58°C 30 seconds
72°C 90 seconds
25 cycles (10 minute extension at 72°C-4°C incubation)

Run 5 microliters on a 1% agarose gel to check the reaction.
Purify on a Qiaquick column (Qiagen).
Resuspend in 50 microliters $H_2O$.

**Restriction Digest**

25 microliters purified PCR product
10 microliters NEB Buffer #2
3 microliters Kpn I (10U/microliter)
3 microliters EcoR1 (20U/microliter)
59 microliters $H_2O$

Cut for 2 hours at 37°C.
Purify on a Qiaquick column (Qiagen).
Elute with 35 microliters $H_2O$.

**Ligation**

10 microliters Digested PCR product
5 microliters pBluescript Vector (cut with
EcoRI/KpnI and phosphatased with shrimp alkaline phosphatase)
4 microliters 5x Ligation Buffer
1 microliter T4 DNA Ligase (BRL)

Ligate overnight.
Transform into M15pREP4 cells using electroporation.
Plate 100 or 200 microliters onto LB amp meth kan plates, grow overnight at 37 degrees celsius.

**Beta-Glycosidase Assay**

Perform glycosidase assay to screen for mutants as follows. The filter assay uses buffer Z (see recipe below) containing 1 mg/ml of the substrate 5-bromo-4-chloro-3-indolyl-β-o-glucopyranoside (XGLU) (Diagnostic Chemicals Limited or Sigma).
Z-Buffer: (referenced in Miller, J.H. (1992) A Short Course in Bacterial Genetics, p. 445.)
per liter:

| | |
|---|---|
| $Na_2HPO_4$-$7H_2O$ | 16.1 g |
| $NaH_2PO_4$-$H_2O$ | 5.5 g |
| KCl | 0.75 g |
| $MgSO_4$-$7H_2O$ | 0.246 g |
| β-mercaptoethanol | 2.7 ml |

Adjust pH to 7.0

(1) Perform colony lifts using Millipore HATF membrane filters.

(2) Lyse colonies with chloroform vapor in 150 mm glass petri dishes.

(3) Transfer filters to 100 mm glass petri dishes containing a piece of Whatman 3MM filter paper saturated with Z buffer containing 1 mg/ml XGLU. After transferring filter bearing lysed colonies to the glass petri dish, maintain dish at room temperature.

(4) "Positives" were observed as blue spots on the filter membranes ("positives" are spots which appear early). Use the following filter rescue technique to retrieve plasmid from lysed positive colony. Use pasteur pipette (or glass capillary tube) to core blue spots on the filter membrane. Place the small filter disk in an Epp tube containing 20 μl water. Incubate the Epp tube at 75°C for 5 minutes followed by vortexing to elute plasmid DNA off filter. Transform this DNA into electrocompetent *E. coli* cells. Repeat filter-lift assay on transformation plates to identify "positives." Return transformation plates to 37°C incubator after filter lift to regenerate colonies. Inoculate 3 ml LBamp liquid with repurified positives and incubate at 37°C overnight. Isolate plasmid DNA from these cultures and sequence plasmid insert.

The β-glycosidase subjected to mutagenesis acted on XGLU 2.5 times more efficiently than wild-type β-glycosidase.

**Claims**

1. A method for obtaining an enzyme activity of interest, said method including the steps of:

    (a) providing a first library with DNA obtained from at least one microorganism;
    (b) binding at least a portion of the coding sequences of the first library to a nucleic acid probe specific for the enzyme activity of interest to create a DNA subset;
    (c) providing a second library which is an expression library using DNA from the DNA subset obtained from step (b); and
    (d) screening the second library for the enzyme activity.

2. The method of claim 1, further including the step of introducing at least one mutation into DNA contained in the first library prior to the binding step.

3. The method of claim 2, further including the step of determining the specific activity of the at least one member of the second library.

4. The method of claim 3, further including the step of comparing the activity of a DNA expression product from the second library with the activity encoded by a non-mutagenized form of the DNA present in the first library, wherein a difference in activity is indicative of an effect of introducing at least one mutation.

5. The method of claim 2, further including the step of obtaining DNA from a clone which is positive for at least one common characteristic which may be the same or different from the enzyme activity of interest, prior to said step of introducing at least one mutation.

6. The method of claim 5, wherein obtaining the DNA contained in the clone positive for the at least one common characteristic includes the steps of contacting the clone with a complementary nucleic acid, or fragment thereof, thereby allowing hybridisation of the clone DNA with the complementary nucleic acid, and isolation of the hybridised clone DNA.

7. The method of claim 6, wherein the complementary nucleic acid or fragment thereof includes a solid-phase-bound hybridisation probe.

8. The method of claim 2, wherein the mutation is introduced by a method selected from error-prone PCR, oligonucleotide-directed mutagenesis, assembly PCR, sexual PCR mutagenesis, in vivo mutagenesis, cassette mutagenesis, recursive ensemble mutagenesis, exponential ensemble mutagenesis and site-specific mutagenesis.

9. The method of claim 2, including the step of screening DNA contained in the first library for an activity which is the same or different from the enzyme activity of interest, prior to subjecting the DNA to mutagenesis.

10. The method of claim 1, wherein the DNA is a pool of DNA populations containing a plurality of desired characteristics and is obtained from a mixed population of microorganisms.

11. The method of claim 1, wherein, before the screening step, the method further includes the steps of:

    (i) providing a first library with genomic DNA obtained from a mixed population of microorganisms;
    (ii) rendering the DNA of step (i) single-stranded;
    (iii) contacting the single-stranded DNA population of (ii) with a DNA probe;
    (iv) releasing from the probe the members of the genomic population which had been bound to the probe;
    (v) forming double-stranded DNA from the members of the genomic population of (iv); and

(vi) introducing the double-stranded DNA of (v) into a suitable host cell to produce an expression library containing a plurality of clones containing the selected DNA.

12. The method of claim 11, wherein the genomic DNA, or fragment thereof, include one or more operons, or portions thereof.

13. The method of claim 12, wherein the operons, or portions thereof, encode a complete or partial metabolic pathway.

14. The method of claim 13, wherein the operons, or portions thereof, which encode a complete or partial metabolic pathway, encode polyketide synthases.

15. The method of claim 11, wherein the DNA probe is bound to a ligand and includes at least a portion of the coding region sequence of DNA for an enzyme of known activity, and wherein a binding partner for said ligand is bound to a solid phase.

16. The method of claim 15, wherein the ligand is selected from antigens; haptens, biotin, iminobiotin, sugars, enzymes, apoenzymes, homopolymeric oligonucleotides and hormones.

17. The method of claim 15, wherein the binding partner for said ligand is selected from antibodies, or specific binding fragments thereof, avidin, streptavidin, lectins, enzyme inhibitors, apoenzymes, cofactors, homopolymeric oligonucleotides and hormone receptors.

18. The method of claim 15, wherein the solid phase is selected from glass surfaces, polymeric surfaces, pack columns of polymeric beads, magnetic particles, and paramagnetic particles.

19. The method of claim 1, wherein, before the screening step, the method further includes the steps of:

(i) obtaining a single-stranded genomic DNA population from DNA contained in the first library;
(ii) contacting the single-stranded DNA population of (i) with a ligand-bound oligonucleotide probe that is complementary to a secretion signal sequence unique to a given class of proteins under conditions permissive of hybridisation to form a double-stranded complex;
(iii) contacting the double-stranded complex of (ii) with a solid-phase-specific binding partner for said ligand so as to produce a solid phase complex;
(iv) separating the solid phase complex from the single-stranded DNA population of (i);
(v) releasing the members of the genomic population which had bound to said solid-phase-bound probe;
(vi) separating the solid-phase-bound probe from the members of the genomic population which had bound thereto;
(vii) forming double-stranded DNA from the members of the genomic population of (v); and
(viii) introducing the double-stranded DNA of (vii) into a suitable host cell to form an expression library containing a plurality of clones containing the selected DNA.

20. The method of claim 19, wherein the genomic DNA, or fragment thereof, include one or more operons, or portions thereof.

21. The method of claim 2, wherein the first library is obtained from DNA contained in a mixed population of microorganisms.

22. A method for obtaining an enzyme activity of interest according to claim 1, wherein in step (a) of claim 1 the DNA of the first library is obtained from a mixture of microorganisms, wherein in step (b) of claim 1 a mixture of DNA probes including at least a portion of a DNA sequence encoding at least one protein related to the enzymatic activity is bound to the DNA of the first library and wherein after having performed steps (c) and (d) of claim 1 a clone is identified which is positive for the enzymatic activity.

23. The method of claim 22, wherein the enzymatic activity is the activity of an enzyme selected from oxidoreductases, transferases, hydrolases, lyases, isomerases, and ligases.

24. The method of claim 22, wherein the DNA of the first library is genomic DNA obtained from one or more species of uncultured microorganisms.

**25.** The method of any one of claims 1-23, wherein the genomic DNA is derived from uncultured microorganisms.

**26.** The method of any one of claims 1-23, wherein the genomic DNA is derived from cultured microorganisms.

**27.** The method of any one of claims 25-26, wherein the microorganisms are isolated from an environmental sample.

**28.** The method of claim 27, wherein the microorganisms isolated from an environmental sample are extremophiles.

**29.** The method of claim 28, wherein the extremophiles are selected from the group consisting of thermophiles, hyperthermophiles, psychrophiles and psychrotrophs.

**30.** The method of any one of claims 1-29, wherein the enzyme having the enzyme activity of interest is a thermostable enzyme.

**31.** The method of any of claims 1-30, wherein the DNA is from a heterogeneous DNA population.

**Patentansprüche**

**1.** Verfahren zur Gewinnung einer Enzymaktivität von Interesse, wobei das Verfahren die Schritte einschließt:

(a) Bereitstellen einer ersten Bibliothek mit DNA, die von mindestens einem Mikroorganismus erhalten wird;
(b) Binden mindestens eines Teils der codierenden Sequenzen der ersten Bibliothek an eine für die Enzymaktivität von Interesse spezifische Nucleinsäuresonde, um eine DNA-Teilmenge zu bilden;
(c) Bereitstellen einer zweiten Bibliothek, die eine Expressionsbibliothek ist, wobei DNA von der in Schritt (b) erhaltenen DNA-Teilmenge verwendet wird; und
(d) Durchmustern der zweiten Bibliothek auf enzymatische Aktivität.

**2.** Verfahren nach Anspruch 1, das weiterhin den Schritt des Einführens mindestens einer Mutation in die in der ersten Bibliothek enthaltenen DNA vor dem Bindungsschritt einschließt.

**3.** Verfahren nach Anspruch 2, das weiterhin den Schritt des Bestimmens der spezifischen Aktivität mindestens eines Mitglieds der zweiten Bibliothek einschließt.

**4.** Verfahren nach Anspruch 3, das weiterhin den Schritt des Vergleichens der Aktivität eines DNA-Expressionsprodukts von der zweiten Bibliothek mit der von einer nicht mutagenisierten Form der in der ersten Bibliothek vorhandenen DNA codierten Aktivität einschließt, wobei der Unterschied der Aktivitäten eine Wirkung der Einführung mindestens einer Mutation anzeigt.

**5.** Verfahren nach Anspruch 2, das weiterhin den Schritt des Gewinnens einer DNA von einem Clon einschließt, der für mindestens ein gemeinsames Merkmal positiv ist, das die gleiche oder eine unterschiedliche Enzymaktivität als die von Interesse sein kann, vor dem Schritt des Einführens mindestens einer Mutation.

**6.** Verfahren nach Anspruch 5, wobei das Gewinnen der in dem Clon enthaltenen DNA, der für mindestens ein gemeinsames Merkmal positiv ist, die Schritte des Inkontaktbringens des Clons mit einer komplementären Nucleinsäure oder einem Fragment davon, wodurch die Hybridisierung der Clon-DNA mit der komplementären Nucleinsäure ermöglicht wird, und das Isolieren der hybridisierten Clon-DNA einschließt.

**7.** Verfahren nach Anspruch 6, wobei die komplementäre Nucleinsäure oder das Fragment davon eine an eine Festphase gebundene Hybridisierungssonde einschließt.

**8.** Verfahren nach Anspruch 2, wobei die Mutation durch ein Verfahren eingeführt wird, das ausgewählt ist aus von fehleranfälliger PCR, oligonucleotidgesteuerter Mutagenese, Assembly-PCR, sexueller PCR-Mutagenese, In-vivo-Mutagenese, Kassetten-Mutagenese, rekursiver Ensemble-Mutagenese, exponentieller Ensemble-Mutagenese und ortspezifischer Mutagenese.

**9.** Verfahren nach Anspruch 2, einschließlich des Schrittes des Durchmusterns der in der ersten Bibliothek enthaltenen DNA auf eine Aktivität, die die gleiche oder eine unterschiedliche Enzymaktivität als die von Interesse ist, bevor die DNA einer Mutagenese unterzogen wird.

10. Verfahren nach Anspruch 1, wobei die DNA ein Pool von DNA-Populationen ist, die eine Vielzahl von gewünschten Eigenschaften enthalten, und wobei die DNA von einer gemischten Population von Mikroorganismen erhalten wird.

11. Verfahren nach Anspruch 1, wobei das Verfahren vor dem Durchmusterungsschritt weiterhin die Schritte einschließt:

(i) Bereitstellen einer ersten DNA-Bibliothek mit genomischer DNA, die von einer gemischten Population aus Mikroorganismen erhalten wird;
(ii) Umwandeln der DNA aus Schritt i) in Einzelstränge;
(iii) Inkontaktbringen der einzelsträngigen DNA-Population aus ii) mit einer DNA-Sonde;
(iv) Freisetzen der Mitglieder der genomischen Population, die an die Sonde gebunden war, von der Sonde;
(v) Bilden doppelsträngiger DNA aus den Mitgliedern der genomischen Population aus (iv); und
(vi) Einführen der doppelsträngigen DNA aus (v) in eine geeignete Wirtszelle, um eine Expressionsbibliothek herzustellen, die eine Vielzahl an Clonen enthält, die die gewählte DNA enthalten.

12. Verfahren nach Anspruch 11, wobei die genomische DNA oder das. Fragment davon ein oder mehrere Operonen oder Teile davon enthalten.

13. Verfahren nach Anspruch 12, wobei die Operonen oder Teile davon einen kompletten oder teilweisen Stoffwechselweg codieren.

14. Verfahren nach Anspruch 13, wobei die Operonen oder Teile davon, die einen kompletten oder teilweisen Stoffwechselweg codieren, Polyketidsynthasen codieren.

15. Verfahren nach Anspruch 11, wobei die DNA-Sonde an einen Liganden gebunden ist und mindestens einen Teil der Sequenz der codierenden Region der DNA für ein Enzym mit bekannter Aktivität einschließt, und wobei ein Bindungspartner für den Liganden an eine Festphase gebunden ist.

16. Verfahren nach Anspruch 15, wobei der Ligand ausgewählt ist aus Antigenen, Haptenen, Biotin, Iminobiotin, Zuckern, Enzymen, Apoenzymen, homopolymeren Oligonucleotiden und Hormonen.

17. Verfahren nach Anspruch 15, wobei der Bindungspartner für den Liganden ausgewählt ist aus Antikörpern oder spezifischen Bindefragmenten davon, Avidin, Streptavidin, Lektinen, Enzyminhibitoren, Apoenzymen, Cofaktoren, homopolymeren Oligonucleotiden und Hormonrezeptoren.

18. Verfahren nach Anspruch 15, wobei die Festphase ausgewählt ist aus Glasoberflächen, Polymeroberflächen, gepackten Säulen von Polymerkügelchen, magnetischen Partikeln und paramagnetischen Partikeln.

19. Verfahren nach Anspruch 1, wobei das Verfahren vor dem Durchmusterungsschritt weiterhin die Schritte einschließt:

(i) Gewinnung einer einzelsträngigen genomischen DNA-Population von der in der ersten Bibliothek enthaltenen DNA;
(ii) Inkontaktbringen der einzelsträngigen DNA-Population aus (i) mit einer an einen Liganden gebundenen Oligonucleotidsonde, die zu einer Sekretionssignalsequenz komplementär ist, die bei einer gegebenen Klasse von Proteinen einzigartig ist, unter Bedingungen, die eine Hybridisierung erlauben, um einen doppelsträngigen Komplex zu bilden;
(iii) Inkontaktbringen des doppelsträngigen Komplexes aus (ii) mit einem für eine Festphase spezifischen Bindungspartner für den Liganden, um einen Festphasenkomplex herzustellen;
(iv) Trennen des Festphasenkomplexes von der einzelsträngigen DNA-Population aus (i);
(v) Freisetzen der Mitglieder der genomischen Population, die an die Sonde gebunden hatten, die an die Festphase gebunden war;
(vi) Trennen der an die Festphase gebundenen Sonde von den Mitgliedern der genomischen Population, die daran gebunden hatten;
(vii) Bilden einer doppelsträngigen DNA aus den Mitgliedern der genomischen Population aus (v); und
(viii) Einführen der doppelsträngigen DNA aus (vii) in eine geeignete Wirtszelle, um eine Expressionsbibliothek zu erzeugen, die eine Vielzahl an Clonen enthält, die die gewählte DNA enthalten.

20. Verfahren nach Anspruch 19, wobei die genomische DNA oder das Fragment davon ein oder mehrere Operone oder Teile davon enthält.

21. Verfahren nach Anspruch 2, wobei die erste Bibliothek von der in einer gemischten Population von Mikroorganismen enthaltenen DNA erhalten wird.

22. Verfahren zur Gewinnung einer Enzymaktivität von Interesse nach Anspruch 1, wobei in Schritt (a) von Anspruch 1 die DNA der ersten Bibliothek aus einem Gemisch aus Mikroorganismen erhalten wird,
    wobei in Schritt (b) von Anspruch 1 ein Gemisch aus DNA-Sonden, die mindestens einen Teil einer DNA-Sequenz einschließen, die mindestens ein mit der Enzymaktivität in Verbindung stehendes Protein codiert, an die DNA der ersten Bibliothek gebunden ist, und wobei nach Ausführung der Schritte (c) und (d) von Anspruch 1 ein Clon identifiziert wird, der für die Enzymaktivität positiv ist.

23. Verfahren nach Anspruch 22, wobei die Enzymaktivität die Aktivität eines Enzyms ist, das ausgewählt ist aus Oxidoreductasen, Transferasen, Hydrolasen, Lyasen, Isomerasen und Ligasen.

24. Verfahren nach Anspruch 22, wobei die DNA der ersten Bibliothek eine genomische DNA ist, die von ein oder mehreren Arten von ungezüchteten Mikroorganismen erhalten wird.

25. Verfahren nach einem der Ansprüche 1 bis 23, wobei die genomische DNA von ungezüchteten Mikroorganismen abstammt.

26. Verfahren nach einem der Ansprüche 1 bis 23, wobei die genomische DNA von gezüchteten Mikroorganismen abstammt.

27. Verfahren nach einem der Ansprüche 25 bis 26, wobei die Mikroorganismen von einer Umweltprobe isoliert werden.

28. Verfahren nach Anspruch 27, wobei die von einer Umweltprobe isolierten Mikroorganismen Extremophile sind.

29. Verfahren nach Anspruch 28, wobei die Extremophile ausgewählt sind aus der Gruppe bestehend aus Thermophilen, Hyperthermophilen, Psychrophilen und Psychrotrophen.

30. Verfahren nach einem der Ansprüche 1 bis 29, wobei das Enzym, das die Enzymaktivität von Interesse hat, ein thermostabiles Enzym ist.

31. Verfahren nach einem der Ansprüche 1 bis 30, wobei die DNA aus einer heterogenen DNA-Population stammt.

**Revendications**

1. Méthode pour obtenir une activité enzymatique d'intérêt, ladite méthode incluant les étapes de :

    (a) fournir une première banque avec de l'ADN obtenu à partir d'au moins un microorganisme;
    (b) lier au moins une portion des séquences codantes de la première banque à une sonde d'acide nucléique spécifique de l'activité enzymatique d'intérêt pour créer un sous-groupe d'ADN ;
    (c) fournir une deuxième banque qui est une banque d'expression utilisant l'ADN du sous-groupe d'ADN obtenu à l'étape (b) ; et
    (d) cribler la deuxième banque pour l'activité enzymatique.

2. Méthode selon la revendication 1, incluant en outre une étape d'introduction d'au moins une mutation dans l'ADN contenu dans la première banque avant l'étape de liaison.

3. Méthode selon la revendication 2, incluant en outre une étape de détermination de l'activité spécifique d'au moins un membre de la deuxième banque.

4. Méthode selon la revendication 3, incluant en outre une étape de comparaison de l'activité d'un produit d'expression d'ADN de la deuxième banque avec l'activité codée par une forme non mutée de l'ADN présent dans la première banque, dans laquelle une différence d'activité est révélatrice d'un effet de l'introduction d'au moins une mutation.

5. Méthode selon la revendication 2, incluant en outre une étape d'obtention de l'ADN d'un clone qui est positif pour au moins une caractéristique commune qui peut être la même ou différente de l'activité enzymatique d'intérêt, avant ladite étape d'introduction d'au moins une mutation.

6. Méthode selon la revendication 5, dans laquelle l'obtention de l'ADN contenu dans le clone positif pour au moins une caractéristique commune inclut les étapes de mise en contact du clone avec un acide nucléique complémentaire, ou un fragment de celui-ci, permettant ainsi l'hybridation de : l'ADN du clone avec l'acide nucléique complémentaire, et d'isolement de l'ADN du clone hybridé.

7. Méthode selon la revendication 6, dans laquelle l'acide nucléique complémentaire ou un fragment de celui-ci inclut une sonde d'hybridation liée à une phase solide.

8. Méthode selon la revendication 2, dans laquelle la mutation est introduite par une méthode sélectionnée parmi une PCR sujette à erreur, une mutagenèse dirigée par un oligonucléotide, une PCR d'assemblage, une mutagenèse par PCR sexuelle, une mutagenèse in vivo, une mutagenèse par cassette, une mutagenèse par ensemble récursif, une mutagenèse par ensemble exponentiel, et une mutagenèse site-spécifique.

9. Méthode selon la revendication 2, incluant une étape de criblage de l'ADN contenu dans la première banque pour une activité qui est la même ou différente de l'activité enzymatique d'intérêt, avant de soumettre l'ADN à la mutagenèse.

10. Méthode selon la revendication 1, dans laquelle l'ADN est un ensemble de populations d'ADN contenant une pluralité de caractéristiques désirées et est obtenu à partir d'une population mélangée de microorganismes.

11. Méthode selon la revendication 1, dans laquelle, avant l'étape de criblage, la méthode inclut en outre les étapes de :

    (i) fournir une première banque d'ADN génomique obtenu à partir d'une population mélangée de microorganismes ;
    (ii) rendre l'ADN de l'étape (i) simple brin ;
    (iii) mettre en contact la population d'ADN simple brin de (ii) avec une sonde d'ADN ;
    (iv) libérer de la sonde les membres de la population génomique qui ont été liés à la sonde;
    (v) former un ADN double brin à partir des membres de la population génomique de (iv) ; et
    (vi) introduire l'ADN double brin de (v) dans une cellule hôte adaptée pour produire une banque d'expression contenant une pluralité de clones contenant l'ADN sélectionné.

12. Méthode selon la revendication 11, dans laquelle l'ADN génomique, ou un fragment de celui-ci, inclut un ou plusieurs opérons, ou des portions de ceux-ci.

13. Méthode selon la revendication 12, dans laquelle les opérons, ou des portions de ceux-ci, codent une voie métabolique complète ou partielle.

14. Méthode selon la revendication 13, dans laquelle les opérons, ou des portions de ceux-ci, qui codent une voie métabolique complète ou partielle, codent pour des polyketide synthases.

15. Méthode selon la revendication 11, dans laquelle la sonde d'ADN est liée à un ligand et inclut au moins une portion de la séquence d'ADN de la région codante pour une enzyme d'activité connue, et dans laquelle un partenaire de liaison pour ledit ligand est lié à une phase solide.

16. Méthode selon la revendication 15, dans laquelle le ligand est sélectionné parmi les antigènes, les haptènes, la biotine, l'iminobiotine, les sucres, les enzymes, les apoenzymes, les oligonucléotides homopolymériques et les hormones.

17. Méthode selon la revendication 15, dans laquelle le partenaire de liaison pour ledit ligand est sélectionné parmi les anticorps, ou des fragments de ceux-ci présentant la spécificité de liaison, l'avidine, la streptavidine, les lectines, les inhibiteurs d'enzyme, les apoenzymes, les cofacteurs, les oligonucléotides homopolymériques et les récepteurs d'hormone.

**18.** Méthode selon la revendication 15, dans laquelle la phase solide est sélectionnée parmi les surfaces de verre, les surfaces polymériques, les colonnes remplies de billes polymériques, les particules magnétiques, et les particules paramagnétiques.

**19.** Méthode selon la revendication 1, dans laquelle, avant l'étape de criblage, la méthode inclut en outre les étapes de :

    (i) obtenir une population d'ADN génomique simple brin à partir de l'ADN contenu dans la première banque ;
    (ii) mettre en contact la population d'ADN simple brin de (i) avec une sonde oligonucléotidique liée à un ligand qui est complémentaire d'une séquence signal de sécrétion unique à une classe donnée de protéines dans des conditions permissives pour l'hybridation de manière à former un complexe double brin ;
    (iii) mettre en contact le complexe double brin de (ii) avec un partenaire de liaison en phase solide spécifique dudit ligand de façon à produire un complexe en phase solide ;
    (iv) séparer le complexe en phase solide de la population d'ADN simple brin de (i);
    (v) libérer les membres de la population génomique qui ont été liés à ladite sonde fixée sur la phase solide ;
    (vi) séparer la sonde fixée à la phase solide des membres de la population génomique qui ont été liés à celle-ci ;
    (vii) former de l'ADN double brin à partir des membres de la population génomique de (v) ; et
    (viii) introduire l'ADN double brin de (vii) dans une cellule hôte adaptée à la préparation d'une banque d'expression contenant une pluralité de clones contenant l'ADN sélectionné.

**20.** Méthode selon la revendication 19, dans laquelle l'ADN génomique, ou un fragment de celui-ci, inclut un ou plusieurs opérons, ou des portions de ceux-ci.

**21.** Méthode selon la revendication 2, dans laquelle la première banque est obtenue à partir d'ADN contenu dans une population mélangée de microorganismes.

**22.** Méthode pour obtenir une activité enzymatique d'intérêt selon la revendication 1, dans laquelle, dans l'étape (a) de la revendication 1, l'ADN de la première banque est obtenu à partir d'un mélange de microorganismes, dans laquelle, dans l'étape (b) de la revendication 1, un mélange de sondes d'ADN incluant au moins une portion d'une séquence d'ADN codant au moins une protéine associée à l'activité enzymatique est lié à l'ADN de la première banque et dans laquelle, après avoir effectué les étapes (c) et (d) de la revendication 1, un clone positif pour l'activité enzymatique est identifié.

**23.** Méthode selon la revendication 22, dans laquelle l'activité enzymatique est l'activité d'une enzyme sélectionnée parmi les oxydoréductases, les transférases, les hydrolases, les lyases, les isomérases et les ligases.

**24.** Méthode selon la revendication 22, dans laquelle l'ADN de la première banque est de l'ADN génomique obtenu à partir d'une ou plusieurs espèces de microorganismes non-cultivés.

**25.** Méthode selon l'une quelconque des revendications 1-23, dans laquelle l'ADN génomique est dérivé de microorganismes non-cultivés.

**26.** Méthode selon l'une quelconque des revendications 1-23, dans laquelle l'ADN génomique est dérivé de microorganismes cultivés.

**27.** Méthode selon l'une quelconque des revendications 25-26, dans laquelle les microorganismes sont isolés d'un échantillon environnemental.

**28.** Méthode selon la revendication 27, dans laquelle les microorganismes isolés d'un échantillon environnemental sont extrêmophiles.

**29.** Méthode selon la revendication 28, dans laquelle les extrêmophiles sont sélectionnés parmi le groupe consistant en les thermophiles, les hyperthermophiles, les psychrophiles et les psychrotrophes.

**30.** Méthode selon l'une quelconque des revendications 1-29, dans laquelle l'enzyme présentant l'activité enzymatique d'intérêt est une enzyme thermostable.

**31.** Méthode selon l'une quelconque des revendications 1-30, dans laquelle l'ADN provient d'une population d'ADN hétérogène.

10 ng (lib 17)
1 ng
100 pg
10 pg

FIG. IA

a
b
c Gene specific primers
d (1:100 dilution)
e
f
standards

1 pg (lib 17)
100 fg
10 fg
1 fg

FIG. IB

a
b
c
d Vector specific primers
e (1:1000 dilution)
f
standards

Panned    Unpanned

FIG. 2

# FIG. 3A

```
  1  ATG CTA CCA GAA GGC TTT CTC TGG GGC GTG
  1  Met Leu Pro Glu Gly Phe Leu Trp Gly Val

 61  GAC AAG CTC AGG AGG AAC ATT GAT CCG AAC
 21  Asp Lys Leu Arg Arg Asn Ile Asp Pro Asn

121  TTC AAC ATA AAG AGG GAA CTC GTC AGC GGC
 41  Phe Asn Ile Lys Arg Glu Leu Val Ser Gly

181  GAA CTT TAC GAG AAG GAT CAC CCC CTC GCC
 61  Glu Leu Tyr Glu Lys Asp His Pro Leu Ala

241  GGA ATA GAG TGG AGC AGG ATC TTT CCC TGG
 81  Gly Ile Glu Trp Ser Arg Ile Phe Pro Trp

301  CGG GAC AGC TAC GGA CTC GTG AAG GAC GTC
101  Arg Asp Ser Tyr Gly Leu Val Lys Asp Val

361  GAC GAG ATA GCG AAT CAT CAG GAG ATA GCC
121  Asp Glu Ile Ala Asn His Gln Glu Ile Ala

421  GAG CTG GGC TTC AAG GTC ATC GTG AAC CTC
141  Glu Leu Gly Phe Lys Val Ile Val Asn Leu

481  GAT CCG ATA ATC GCG AGG GAG AAG GCC CTC
161  Asp Pro Ile Ile Ala Arg Glu Lys Ala Leu

541  GAG AGC GTG GTG GAG TTC GCC AAG TAC GCG
181  Glu Ser Val Val Glu Phe Ala Lys Tyr Ala
```

← Match with FIG. 3 A

# FIG. 3B

```
TCC CAG TCC GGC TTT CAG TTC GAG ATG GGC      60
Ser Gln Ser Gly Phe Gln Phe Glu Met Gly      20

ACA GAC TGG TGG AAG TGG GTC AGG GAT CCC     120
Thr Asp Trp Trp Lys Trp Val Arg Asp Pro      40

GAC CTG CCC GAG GAG GGG ATA AAC AAC TAC     180
Asp Leu Pro Glu Glu Gly Ile Asn Asn Tyr      60

AGA GAC CTC GGT CTG AAC GTT TAC AGG ATT     240
Arg Asp Leu Gly Leu Asn Val Tyr Arg Ile      80

CCA ACG TGG TTT GTG GAG GTT GAC GTT GAA     300
Pro Thr Trp Phe Val Glu Val Asp Val Glu     100

AAA ATC GAT AAA GAC ACG CTC GAA GAG CTC     360
Lys Ile Asp Lys Asp Thr Leu Glu Glu Leu     120

TAC TAC CGC CGC GTT ATA GAG CAC CTC AGG     420
Tyr Tyr Arg Arg Val Ile Glu His Leu Arg     140

AAC CAC TTC ACG CTC CCC CTC TGG CTT CAC     480
Asn His Phe Thr Leu Pro Leu Trp Leu His     160

ACC AAC GGT AGG ATT GGC TGG GTC GGG CAG     540
Thr Asn Gly Arg Ile Gly Trp Val Gly Gln     180

GCG TAC ATC GCG AAC GCA CTC GGG GAC CTC     600
Ala Tyr Ile Ala Asn Ala Leu Gly Asp Leu     200
```

MATCH WITH FIG. 3D

Match with FIG. 3 A
FIG. 3C                    Match with FIG. 3D →

601  GTT GAT ATG TGG AGC ACC TTC AAC GAR CCG
201  Val Asp Met Trp Ser Thr Phe Asn Glu Pro

661  CCC TAC TCC GGY TTT CCN CCG GGG GTT ATG
221  Pro Tyr Ser Gly Phe Pro Pro Gly Val Met

721  AAC ATG ATA AAC GCC CAC GCA CTG GCC TAC
241  Asn Met Ile Asn Ala His Ala Leu Ala Tyr

781  GCC GAT AAG GAT TCC CGC TCC GAG GCC GAG
261  Ala Asp Lys Asp Ser Arg Ser Glu Ala Glu

841  NCC TAT CCA NAC GAC TCC AAC GAC CCN AAG
281  Xxx Tyr Pro Xxx Asp Ser Asn Asp Pro Lys

901  TTC CAC AGC GGG CTC TTC TTC GAC GCA ATC
301  Phe His Ser Gly Leu Phe Phe Asp Ala Ile

961  GGT GAG ACC TTC GTC AAA GTT CGG CAT CTC
321  Gly Glu Thr Phe Val Lys Val Arg His Leu

1021 TAC ACG AGA GAA GTC GTC AGG TAT TCG GAG
341  Tyr Thr Arg Glu Val Val Arg Tyr Ser Glu

1081 TTC CGG GGA GTT CAC AAC TAC GGT TAC GCC
361  Phe Arg Gly Val His Asn Tyr Gly Tyr Ala

1141 AGG CCC GTA AGC GAC ATC GGC TGG GAG ATC
381  Arg Pro Val Ser Asp Ile Gly Trp Glu Ile

Match with FIG. 3E

```
ATG GTC GTT GTG GAN CTC GGT TAC CTC GCG    660
Met Val Val Val Xxx Leu Gly Tyr Leu Ala    220


AAC CCC GAG GCG GMN AAN CTG GCA ATC CTC    720
Asn Pro Glu Ala Xxx Xxx Leu Ala Ile Leu    240


AAG ATG ATA AAG AAG TTC GAC AGG GTA AAG    780
Lys Met Ile Lys Lys Phe Asp Arg Val Lys    260


GTC GGG ATA ATC TAC AAC AAC ATA GGC GTT    840
Val Gly Ile Ile Tyr Asn Asn Ile Gly Val    280


GAC GTG AAA NCT NCA GAA AAC GAC AAC TAC    900
Asp Val Lys Xxx Xxx Glu Asn Asp Asn Tyr    300


CAC AAG GGC AAG CTC AAC ATC GAG TTC GAC    960
His Lys Gly Lys Leu Asn Ile Glu Phe Asp    320


AGG GGG AAC GAC TGG ATA GGC GTT AAC TAC   1020
Arg Gly Asn Asp Trp Ile Gly Val Asn Tyr    340


CCC AAG TTC CCG AGC ATA CCC CTG ATA TCC   1080
Pro Lys Phe Pro Ser Ile Pro Leu Ile Ser    360


TGC AGG CCC GGG AGT TCT TCC GCC GAC GGA   1140
Cys Arg Pro Gly Ser Ser Ser Ala Asp Gly    380


TAT CCG GAG GGG ATC TAC GAC TCG ATA AGA   1200
Tyr Pro Glu Gly Ile Tyr Asp Ser Ile Arg    400
```

MATCH WITH FIG. 3F

MATCH WITH FIG. 3C

# F I G. 3 E

MATCH WITH FIG. 3F

```
1201    GAG GCC AAC AAA TAC GGG GTC CCG GTT TAC
 401    Glu Ala Asn Lys Tyr Gly Val Pro Val Tyr


1261    GAC ACC CTG CGG CCG TAC TAC CTC GCG AGC
 421    Asp Thr Leu Arg Pro Tyr Tyr Leu Ala Ser


1321    GCG GGT TAC GAC GTC AGG GGC TAC CTC TAC
 441    Ala Gly Tyr Asp Val Arg Gly Tyr Leu Tyr


1381    CTC GGT TTC AGG ATG AGG TTC GGC CTC TAT
 461    Leu Gly Phe Arg Met Arg Phe Gly Leu Tyr


1441    CCG CGG GAG GAA AGC GTA AAG GTT TAT AGG
 481    Pro Arg Glu Glu Ser Val Lys Val Tyr Arg


1501    GAA ATC CGG GAG AAG TTC GGA CTT GGG TGA
 501    Glu Ile Arg Glu Lys Phe Gly Leu Gly End
```

| | |
|---|---|
| FIG.3A | FIG.3B |
| FIG.3C | FIG.3D |
| FIG.3E | FIG.3F |

# F I G. 3

Match with FIG. 3D

FIG. 3F

← Match with FIG. 3E

```
GTC ACC GAA AAC GGA ATA GCC GAT TCA ACT    1260
Val Thr Glu Asn Gly Ile Ala Asp Ser Thr     420

CAT GTA GCG AAG ATT GAG GAG GCG TAC GAG    1320
His Val Ala Lys Ile Glu Glu Ala Tyr Glu     440

TGG GCG CTG ACC GAC AAC TAC GAG TGG GCC    1380
Trp Ala Leu Thr Asp Asn Tyr Glu Trp Ala     460

AAA GTG GAT CTC ATA ACC AAG GAG AGA ACA    1440
Lys Val Asp Leu Ile Thr Lys Glu Arg Thr     480

GGC ATC GTG GAG AAC AAC GGA GTG AGC AAG    1500
Gly Ile Val Glu Asn Asn Gly Val Ser Lys     500

1530
510
```